(19) Europäisches Patentamt
European Patent Office
Office européen des brevets

(11) Veröffentlichungsnummer: 0 335 156
A1

# (12) EUROPÄISCHE PATENTANMELDUNG

(21) Anmeldenummer: 89104340.8

(22) Anmeldetag: 11.03.89

(51) Int. Cl.⁴: C07D 231/38 , C07D 231/40 , C07D 231/16 , A01N 43/56

(30) Priorität: 26.03.88 DE 3810382

(43) Veröffentlichungstag der Anmeldung: 04.10.89 Patentblatt 89/40

(84) Benannte Vertragsstaaten: BE CH DE FR GB IT LI NL

(71) Anmelder: BAYER AG
D-5090 Leverkusen 1 Bayerwerk(DE)

(72) Erfinder: Schallner, Otto, Dr.
Noldeweg 22
D-4019 Monheim(DE)
Erfinder: Stetter, Jörg, Dr.
Gellertweg 4
D-5600 Wuppertal 1(DE)
Erfinder: Gehring, Reinhold, Dr.
Dasnoeckel 49
D-5600 Wuppertal 11(DE)
Erfinder: Marhold, Albrecht, Dr.
Carl-Duisberg-Strasse 329
D-5090 Leverkusen 1(DE)
Erfinder: Santel, Hans-Joachim, Dr.
Grünstrasse 9 a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Unterdorfstrasse 6 a
D-4000 Düsseldorf 31(DE)

## (54) 5-Amino-1-phenylpyrazole.

(57) Die Erfindung betrifft neue 5-Amino-1-phenylpyrazole der Formel (I),

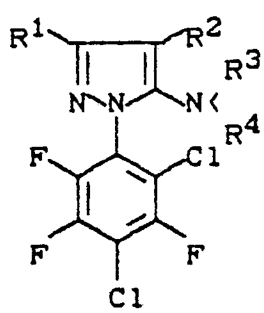

(I)

EP 0 335 156 A1

in welcher
$R^1$ für Wasserstoff oder Alkyl steht,
$R^2$ für Wasserstoff, Nitro, Hydroxycarbonyl oder Alkoxycarbonyl steht,
$R^3$ für Wasserstoff, Alkyl, Alkenyl, Alkinyl, Halogenalkyl, Halogenalkenyl, Halogenalkinyl oder für einen Rest

$$-\underset{\underset{X}{\|}}{C}-R^5$$

steht,
$R^4$ für Wasserstoff, Alkyl, Alkenyl, Alkinyl, Halogenalkyl, Halogenalkenyl, Halogenalkinyl oder für einen Rest

$$-\underset{\underset{X}{\|}}{C}-R^5$$

steht, wobei
$R^5$ für Wasserstoff, Alkyl, Alkenyl, Alkinyl, Halogenalkyl, Halogenalkenyl, Alkoxyalkyl, Alkylthioalkyl, Alkylsulfonylalkyl, Alkylsulfinylalkyl, für gegebenenfalls substituiertes Cycloalkyl, für jeweils gegebenenfalls substituiertes Aryloxyalkyl oder Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und
X für Sauerstoff oder Schwefel steht,
mehrere Verfahren sowie neue Zwischenprodukte zu deren Herstellung und ihre Verwendung als Herbizide.

## 5-Amino-1-phenylpyrazole

Die Erfindung betrifft neue 5-Amino-1-phenylpyrazole, mehrere Verfahren zu ihrer Herstellung und ihre Verwendung als Herbizide.

Es ist bekannt, daß bestimmte 5-Amino-1-phenylpyrazole, wie beispielsweise das 5-Amino-4-nitro-1-(2-chlor-6-fluor-4-trifluormethyl-phenyl)-pyrazol herbizide, insbesondere auch selektiv-herbizide Eigenschaften besitzen (vergl. z.B. DE-OS 34 02 308).

Deren herbizide Wirkung gegenüber Schadpflanzen ist jedoch ebenso wie ihre Verträglichkeit gegenüber wichtigen Kulturpflanzen nicht immer in allen Anwendungsbereichen völlig zufriedenstellend.

Es wurden neue 5-Amino-1-phenylpyrazole der allgemeinen Formel (I),

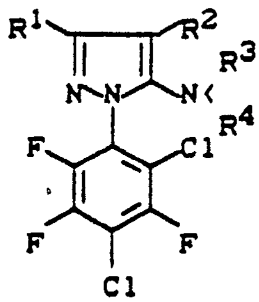

(I)

in welcher

$R^1$ für Wasserstoff oder Alkyl steht,

$R^2$ für Wasserstoff, Nitro, Hydroxycarbonyl oder Alkoxycarbonyl steht,

$R^3$ für Wasserstoff, Alkyl, Alkenyl, Alkinyl, Halogenalkyl, Halogenalkenyl, Halogenalkinyl oder für einen Rest $-\underset{\underset{X}{\|}}{C}-R^5$ steht,

$R^4$ für Wasserstoff, Alkyl, Alkenyl, Alkinyl, Halogenalkyl, Halogenalkenyl, Halogenalkinyl oder für einen Rest $-\underset{\underset{X}{\|}}{C}-R^5$ steht, wobei

$R^5$ für Wasserstoff, Alkyl, Alkenyl, Alkinyl, Halogenalkyl, Halogenalkenyl, Alkoxyalkyl, Alkylthioalkyl, Alkylsulfonylalkyl, Alkylsulfinylalkyl, für gegebenenfalls substituiertes Cycloalkyl, für jeweils gegebenenfalls substituiertes Aryloxyalkyl oder Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und

X für Sauerstoff oder Schwefel steht,

gefunden.

Weiterhin wurde gefunden, daß man die neuen 5-Amino-1-phenylpyrazole der allgemeinen Formel (I),

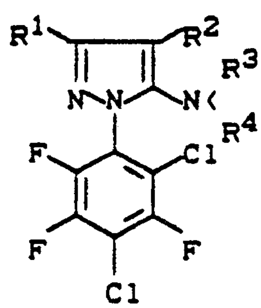

(I)

in welcher

$R^1$ für Wasserstoff oder Alkyl steht,

$R^2$ für Wasserstoff, Nitro, Hydroxycarbonyl oder Alkoxycarbonyl steht,
$R^3$ für Wasserstoff, Alkyl, Alkenyl, Alkinyl, Halogenalkyl, Halogenalkenyl, Halogenalkinyl oder für einen Rest
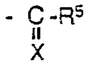
steht,
$R^4$ für Wasserstoff, Alkyl, Alkenyl, Alkinyl, Halogenalkyl, Halogenalkenyl, Halogenalkinyl oder für einen Rest
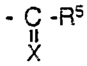
steht, wobei
$R^5$ für Wasserstoff, Alkyl, Alkenyl, Alkinyl, Halogenalkyl, Halogenalkenyl, Alkoxyalkyl, Alkylthioalkyl, Alkylsulfonylalkyl, Alkylsulfinylalkyl, für gegebenenfalls substituiertes Cycloalkyl, für jeweils gegebenenfalls substituiertes Aryloxyalkyl oder Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und
X für Sauerstoff oder Schwefel steht,
nach folgenden Verfahren herstellen kann:
(a) Man erhält 5-Amino-1-phenylpyrazole der Formel (Ia),

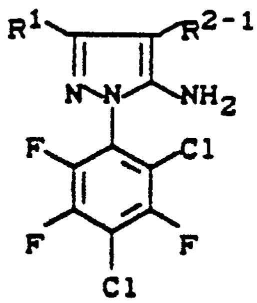
(Ia)

in welcher
$R^{2-1}$ für Wasserstoff, Alkoxycarbonyl oder Nitro steht und
$R^1$ die oben angegebenen Bedeutung hat,
wenn man 2,4-Dichlor-3,5,6-trifluorphenylhydrazin der Formel (II)

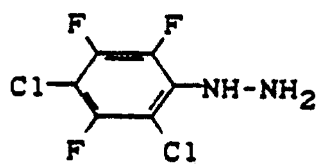
(II)

($\alpha$) mit Acrylnitril-Derivaten der Formel (IIIa),

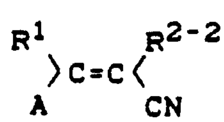
(IIIa)

in welcher
$R^{2-2}$ für Alkoxycarbonyl oder Nitro steht,
A für Halogen, Hydroxy, Alkoxy, Alkylamino oder Dialkylamino steht und
$R^1$ die oben angegebene Bedeutung hat oder
($\beta$) mit 2-Chloracrylnitrilen der Formel (IIIb),

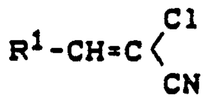

(IIIb)

in welcher.
$R^1$ die oben angegebene Bedeutung hat,
zunächst in einer 1. Stufe gegebenenfalls in Gegenwart eines Verdünnungsmittels und gegebenenfalls in Gegenwart eines Reaktionshilfsmittels umsetzt zu den Phenylhydrazinderivaten der Formel (IV),

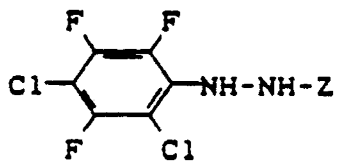

(IV)

in welcher
Z für einen Rest

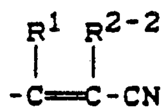

oder für einen Rest

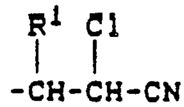

steht,

und diese in einer 2. Stufe gegebenenfalls in Gegenwart eines Verdünnungsmittels und gegebenenfalls in Gegenwart eines Reaktionshilfsmittels cyclisiert;

(b) man erhält 5-Amino-1-phenylpyrazole der Formel (Ib),

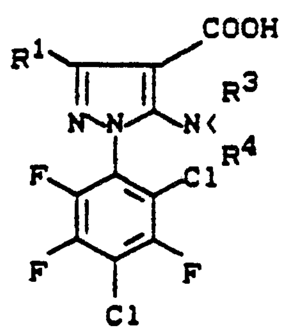

(Ib)

in welcher
$R^1$, $R^3$ und $R^4$ die oben angegebene Bedeutung haben,
wenn man 5-Amino-1-phenylpyrazole der Formel (Iz),

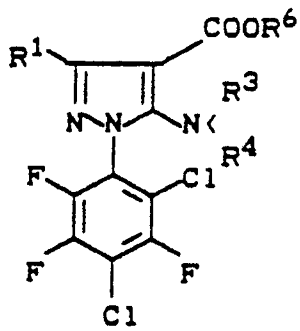

(Iz)

in welcher
$R^6$ für Alkyl steht und
$R^1$, $R^3$ und $R^4$ die oben angegebene Bedeutung haben,
an der Estergruppe in 4-Position des Pyrazolringes in allgemein üblicher Art und Weise gegebenenfalls in Gegenwart eines Verdünnungsmittels und gegebenenfalls in Gegenwart eines Reaktionshilfsmittels verseift;

(c) man erhält 5-Amino-1-phenylpyrazole der Formel (Ic),

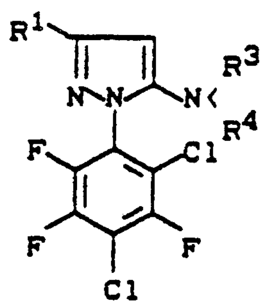

(Ic)

in welcher
$R^1$, $R^3$ und $R^4$ die oben angegebene Bedeutung haben,
wenn man 5-Amino-1-phenylpyrazole der Formel (Ib),

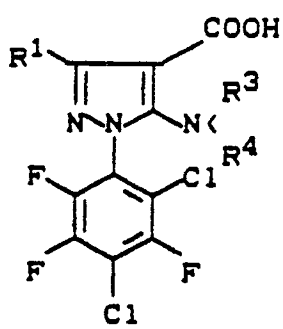

(Ib)

in welcher
$R^1$, $R^3$ und $R^4$ die oben angegebene Bedeutung haben,
in allgemein üblicher Art und Weise gegebenenfalls in Gegenwart eines Verdünnungsmittels und gegebenenfalls in Gegenwart eines Katalysators decarboxyliert;

(d) man erhält 5-Amino-1-phenylpyrazole der Formel (Id),

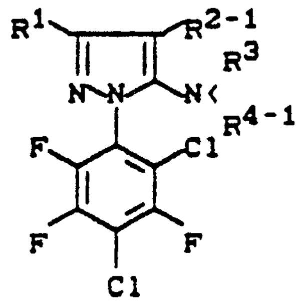

(Id)

in welcher

$R^{2-1}$ für Wasserstoff, Alkoxycarbonyl oder Nitro steht,

$R^{4-1}$ für Alkyl, Alkenyl, Alkinyl, Halogenalkyl, Halogenalkenyl, Halogenalkinyl oder für einen Rest $-\underset{\underset{X}{\parallel}}{C}-R^5$ steht und

$R^1$, $R^3$, $R^5$ und X die oben angegebene Bedeutung haben,

wenn man 5-Amino-1-phenylpyrazole der Formel (Iy),

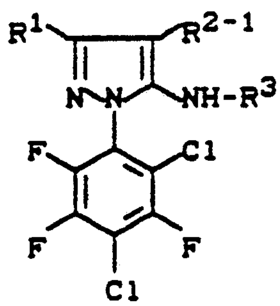

(Iy)

in welcher

$R^1$, $R^{2-1}$ und $R^3$ die oben angegebene Bedeutung haben,

(α) mit Acylierungsmitteln der Formel (V),

$R^5-\overset{\overset{X}{\parallel}}{C}-A^1$   (V)

in welcher

$A^1$ für eine elektronenanziehende Abgangsgruppe steht und

$R^5$ und X die oben angegebene Bedeutung haben, oder

(β) mit Alkylierungsmitteln der Formel (VI),

$R^{4-2}-A^2$   (VI)

in welcher

$R^{4-2}$ für Alkyl, Alkenyl, Alkinyl, Halogenalkyl, Halogenalkenyl oder Halogenalkinyl steht und

$A^2$ für eine elektronenanziehende Abgangsgruppe steht,

jeweils gegebenenfalls in Gegenwart eines Verdünnungsmittels und gegebenenfalls in Gegenwart eines Reaktionshilfsmittels umsetzt;

(e) man erhält 5-Amino-1-phenylpyrazole der Formel (Ie),

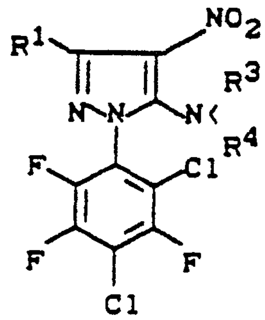

(Ie)

in welcher
$R^1$, $R^3$ und $R^4$ die oben angegebene Bedeutung haben,
wenn man 5-Amino-1-phenylpyrazole der Formel (Ic),

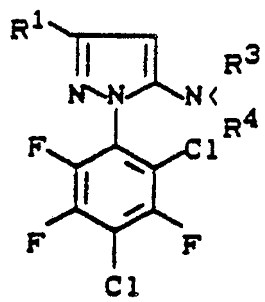

(Ic)

in welcher
$R^1$, $R^3$ und $R^4$ die oben angegebene Bedeutung haben,
mit Nitrierungsmitteln gegebenenfalls in Gegenwart eines Verdünnungsmittels und gegebenenfalls in Gegenwart eines Reaktionshilfsmittels umsetzt;

(f) man erhält 5-Amino-1-phenylpyrazole der Formel (If),

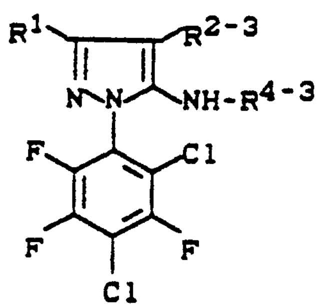

(If)

in welcher
$R^{2-3}$ für Wasserstoff oder Nitro steht,
$R^{4-3}$ für Wasserstoff, Alkyl, Alkenyl, Alkinyl, Halogenalkyl, Halogenalkenyl oder Halogenalkinyl steht und
$R^1$ die oben angegebene Bedeutung hat,
wenn man 5-Amino-1-phenylpyrazole der Formel (Ix),

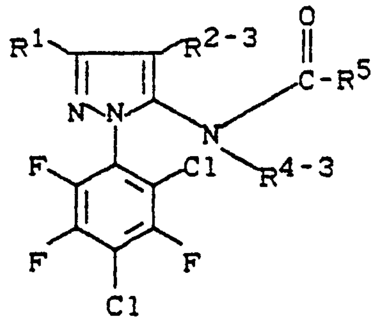

(Ix)

in welcher
$R^1$, $R^{2-3}$, $R^{4-3}$ und $R^5$ die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes deacyliert;

(g) man erhält 5-Amino-1-phenylpyrazole der Formel (Ig),

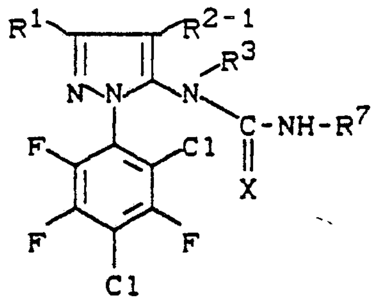

(Ig)

in welcher
$R^{2-1}$ für Wasserstoff, Alkoxycarbonyl oder Nitro steht,
$R^7$ für Alkyl oder für gegebenenfalls substituiertes Aryl steht und
$R^1$, $R^3$ und X die oben angegebene Bedeutung haben,
wenn man 5-Amino-1-phenylpyrazole der Formel (Iy),

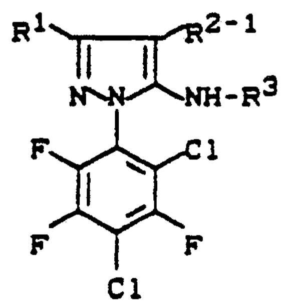

(Iy)

in welcher
$R^1$, $R^{2-1}$ und $R^3$ die oben angegebene Bedeutung haben,
mit Iso(thio)cyanaten der Formel (VII),

$R^7$-N=C=X (VII)

in welcher
$R^7$ und X die oben angegebene Bedeutung haben,
gegebenenfalls in Gegenwart eines Verdünnungsmittels und gegebenenfalls in Gegenwart eines Reaktionshilfsmittels umsetzt;

(h) man erhält 5-Amino-1-phenylpyrazole der Formel (Ih),

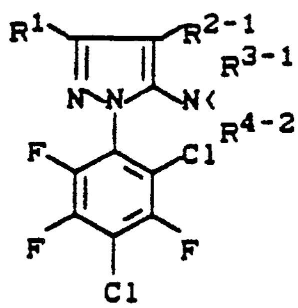

(Ih)

in welcher
$R^{2-1}$ für Wasserstoff, Alkoxycarbonyl oder Nitro steht,
$R^{3-1}$ für Wasserstoff, Alkyl, Alkenyl, Alkinyl, Halogenalkyl, Halogenalkenyl oder Halogenalkinyl steht,
$R^{4-2}$ für Alkyl, Alkenyl, Alkinyl, Halogenalkyl, Halogenalkenyl oder Halogenalkinyl steht und
$R^1$ die oben angegebene Bedeutung hat,
wenn man 5-Halogenpyrazole der Formel (VIII),

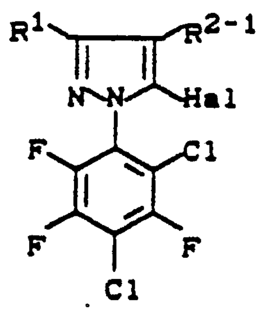

(VIII)

in welcher
Hal für Halogen steht und
$R^1$ und $R^{2-1}$ di oben angegebene Bedeutung haben,
mit Aminen der Formel (IX),

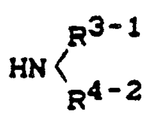

(IX)

in welcher
$R^{3-1}$ und $R^{4-2}$ die oben angegebene Bedeutung haben,
gegebenenfalls in Gegenwart eins Verdünnungsmittels und gegebenenfalls in Gegenwart eines Reaktionshilfsmittels umsetzt;

(i) man erhält 5-Amino-1-phenylpyrazole der Formel (Ii),

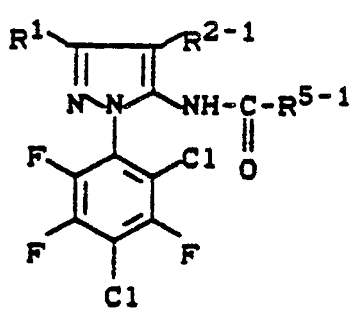

(Ii)

in welcher
$R^{2-1}$ für Wasserstoff, Alkoxycarbonyl oder Nitro steht,
$R^{5-1}$ für Alkoxy, Alkylthio, Alkylamino oder Dialkylamino oder für jeweils gegebenenfalls substituiertes Aryloxy, Arylthio oder Arylamino steht und
$R^1$ die oben angegebene Bedeutung hat,
wenn man (Bis)Carbamate der Formel (Iw),

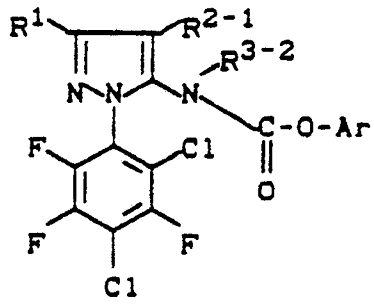

(Iw)

in welcher
$R^{3-2}$ für Wasserstoff oder für einen Rest $-\underset{\underset{O}{\|}}{C}-O-Ar$
steht, wobei
Ar für gegebenenfalls substituiertes Aryl steht und
$R^1$ und $R^{2-1}$ die oben angegebene Bedeutung haben,
mit Alkoholen, Aminen oder Thiolen der Formel (X),
$R^{5-1}$-H (X)
in welcher
$R^{5-1}$ die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen 5-Amino-1-phenyl-pyrazole der allgemeinen Formel (I) herbizide, insbesondere auch selektiv-herbizide Eigenschaften besitzen.

Überraschenderweise zeigen die erfindungsgemäßen 5-Amino-1-phenyl-pyrazole der allgemeinen Formel (I) bei vergleichbar guter allgemein-herbizider Wirksamkeit gegenüber Schadpflanzen eine erheblich verbesserte Verträglichkeit gegenüber wichtigen Kulturpflanzen im Vergleich zu den aus dem Stand der Technik bekannten 5-Amino-1-aryl-pyrazolen, wie beispielsweise die Verbindung 5-Amino-4-nitro-1-(2-chlor-6-fluor-4-trifluormethylphenyl)-pyrazol, welche chemisch und wirkungsgemäß naheliegende Verbindungen sind.

Die erfindungsgemäßen 5-Amino-1-phenylpyrazole sind durch die Formel (I) allgemein definiert. Bevorzugt sind Verbindungen der Formel (I), bei welchen
$R^1$ für Wasserstoff oder für geradkettiges oder verzweigtes Alkyl mit 1 bis 6 Kohlenstoffatomen steht,
$R^2$ für Wasserstoff, Nitro, Hydroxycarbonyl oder geradkettiges oder verzweigtes Alkoxycarbonyl mit 1 bis 6 Kohlenstoffatomen steht,
$R^3$ für Wasserstoff, für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschidenen Halogenatomen oder für einen Rest $-\underset{\underset{X}{\|}}{C}-R^5$
steht und
$R^4$ für Wasserstoff, für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen oder für einen Rest $-\underset{\underset{X}{\|}}{C}-R^5$

steht, wobei

$R^5$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 6 Kohlenstoffatomen, für jeweils geradkettiges oder verzweigtes Halogenalkyl mit 1 bis 4 Kohlenstoffatomen und 1 bis 9 gleichen oder verschiedenen Halogenatomen oder Halogenalkenyl mit 2 bis 6 Kohlenstoffatomen und 1 bis 9 gleichen oder verschiedenen Halogenatomen oder für jeweils geradkettiges oder verzweigtes Alkoxyalkyl, Alkylthioalkyl, Alkylsulfonylalkyl, Alkylsulfinylalkyl, Alkoxy, Alkylthio, Alkylamino oder Dialkylamino mit jeweils 1 bis 4 Kohlenstoffatomen in den einzelnen Alkylteilen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenoxymethyl, Phenoxyethyl, Phenylthio oder Phenylamino steht, wobei als Phenylsubstituenten jeweils in Frage kommen: Halogen, jeweils geradkettiges oder verzweigtes Alkyl oder Alkoxy mit je weils 1 bis 4 Kohlenstoffatomen oderHalogenalkyl mit 1 bis 4 Kohlenstoffatomen und 1 bis 9 gleichen oder verschiedenen Halogenatomen und

X für Sauerstoff oder Schwefel steht.

Besonders bevorzugt sind Verbindungen der Formel (I), bei welchen

$R^1$ für Wasserstoff, Methyl, Ethyl, n- oder i-Propyl sowie n-, i-, s- oder t-Butyl steht,

$R^2$ für Wasserstoff, Nitro, Hydroxycarbonyl, Methoxycarbonyl oder Ethoxycarbonyl steht,

$R^3$ für Wasserstoff, Methyl, Ethyl, n- oder i-Propyl, n-, i-, s- oder t-Butyl, für Allyl, n- oder i-Butenyl, Propargyl, n- oder i-Butinyl, für Chlorethyl, Trifluorethyl, Bromethyl, für Chlorallyl, Dichlorallyl, Brompropargyl oder für einen Rest $-\underset{\underset{X}{\|}}{C}-R^5$

steht und

$R^4$ für Wasserstoff, Methyl, Ethyl, n- oder i-Propyl, n-, i-, s- oder t-Butyl, für Allyl, n- oder i-Butenyl, Propargyl, n- oder i-Butinyl, für Chlorethyl, Trifluorethyl, Bromethyl, für Chlorallyl, Dichlorallyl, Brompropargyl oder für einen Rest $-\underset{\underset{X}{\|}}{C}-R^5$

steht, wobei

$R^5$ für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Undecyl, Vinyl, Allyl, Chlorallyl, Propargyl, Butenyl, Methoxymethyl, Ethoxymethyl, Methoxyethyl, Ethoxyethyl, Methylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methoxy, Ethoxy, n- oder i-Propoxy, Methylthio, Ethylthio, Methylamino, Ethylamino, Dimethylamino, Diethylamino, Trifluormethyl, Trichlormethyl, Dichlorfluormethyl, Difluorchlormethyl, Chlormethyl, Iodmethyl, Brommethyl, Dichlormethyl, Difluormethyl, 1-Chlorethyl, 2-Chlorethyl, 1-Bromethyl, 2-Bromethyl, 1-Chlorpropyl, 3-Chlorpropyl, Heptafluorpropyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl oder für jeweils gegebenenfalls ein- bis dreifach, gleich oder verschieden durch Methyl, Methoxy, Fluor, Chlor oder Trifluormethyl substituiertes Phenyl, Phenoxymethyl, Phenoxy, Phenylthio oder Phenylamino steht und

X für Sauerstoff und Schwefel steht.

Ganz besonders bevorzugt sind Verbindungen der Formel (I), bei welchen

$R^1$ für Wasserstoff oder Methyl steht,

$R^2$ für Wasserstoff, Ethoxycarbonyl oder Nitro steht,

$R^3$ für Wasserstoff, Methyl, Ethyl, n- oder i-Propyl, n-, i-, s- oder t-Butyl, für Allyl, Chlorallyl oder Propargyl steht und

$R^4$ für Wasserstoff, Methyl, Ethyl, n- oder i-Propyl, n- i-, s- oder t-Butyl, für Allyl, Chlorallyl oder Propargyl oder für einen Rest $-\underset{\underset{O}{\|}}{C}-R^5$

steht, wobei

$R^5$ für Wasserstoff, Methyl, Ethyl, n- oder i-Propyl, n-, i-, s- oder t-Butyl, für Chlormethyl, Dichlormethyl, Trichlormethyl, Difluormethyl, Trifluormethyl, Dichlorfluormethyl, Difluorchlormethyl, Brommethyl, für 1-Chlorethyl, 2-Chlorethyl, 1-Bromethyl, 2-Bromethyl, 1-Chlorpropyl, 3-Chlorpropyl, Heptafluor-n-propyl, für Methoxymethyl, Ethoxymethyl, Methoxyethyl, Ethoxyethyl, Methylthiomethyl, Methylsulfonylmethyl, für Methoxy, Ethoxy, n-oder i-Propoxy, Methylamino, Ethylamino, Dimethylamino, Diethylamino, für Cyclopropyl, Cyclopentyl, Cyclohexyl oder für gegebenenfalls ein- bis dreifach, gleich oder verschieden substituiertes Phenoxymethyl steht, wobei

als Substituenten infrage kommen: Fluor, Chlor, Methyl oder Trifluormethyl.

Im einzelnen seien außer den bei den Herstellungsbeispielen genannten Verbindungen die folgenden 5-Amino-1-phenylpyrazole der allgemeinen Formel (I) genannt:

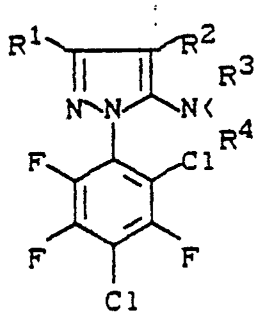

(I)

| $R^1$ | $R^2$ | $R^3$ | $R^4$ |
|---|---|---|---|
| H | $NO_2$ | H | 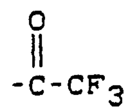 |
| H | $NO_2$ | H | 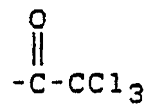 |
| H | $NO_2$ | H | 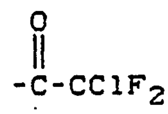 |
| H | $NO_2$ | H | 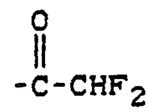 |
| H | $NO_2$ | H | 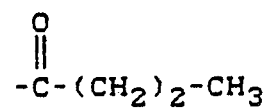 |
| H | $NO_2$ | H | 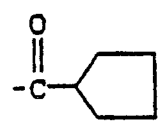 |

| $R^1$ | $R^2$ | $R^3$ | $R^4$ |
|---|---|---|---|
| H | $NO_2$ | H | 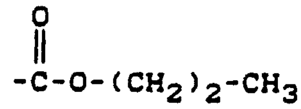 |
| H | $NO_2$ | H | 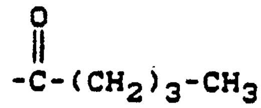 |
| H | $NO_2$ | H | 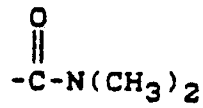 |
| H | $NO_2$ | H | 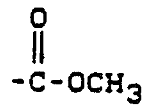 |
| H | $NO_2$ | H | 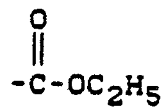 |
| H | $NO_2$ | H | 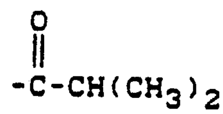 |
| H | $NO_2$ | H | 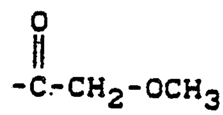 |
| H | $NO_2$ | H | 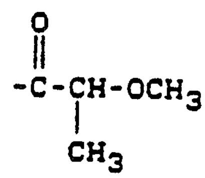 |
| H | $NO_2$ | H | 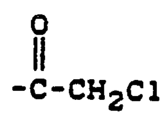 |
| H | $NO_2$ | H | 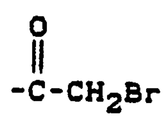 |
| H | $NO_2$ | H | 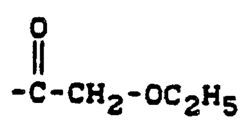 |

| $R^1$ | $R^2$ | $R^3$ | $R^4$ |
|---|---|---|---|
| H | $NO_2$ | H | 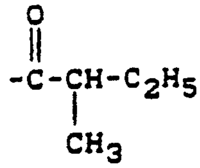 |
| H | $NO_2$ | H | 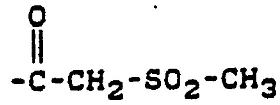 |
| H | $NO_2$ | H | 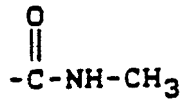 |
| H | $NO_2$ | H | 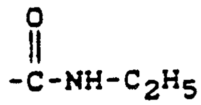 |
| H | $NO_2$ | H | 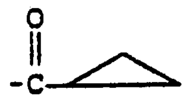 |
| H | $NO_2$ | H | 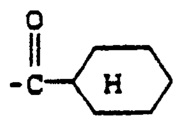 |
| H | $NO_2$ | H | 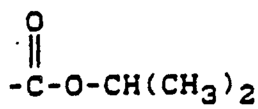 |
| H | $NO_2$ | H | 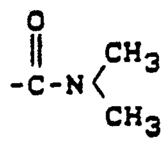 |
| H | H | H | 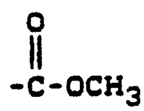 |
| H | H | H | 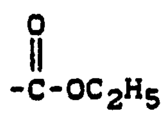 |
| H | H | H | 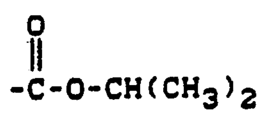 |

| $R^1$ | $R^2$ | $R^3$ | $R^4$ |
|---|---|---|---|
| H | H | H | $-\overset{O}{\overset{\|}{C}}-CH(CH_3)_2$ |
| H | H | H | $-\overset{O}{\overset{\|}{C}}-CH_2-OCH_3$ |
| H | H | H | $-\overset{O}{\overset{\|}{C}}-\underset{\underset{CH_3}{\|}}{CH}-OCH_3$ |
| H | H | H | $-\overset{O}{\overset{\|}{C}}-CH_2-OC_2H_5$ |
| H | H | H | $-\overset{O}{\overset{\|}{C}}-CH_2Cl$ |
| H | H | H | $-\overset{O}{\overset{\|}{C}}-CH_2Br$ |
| H | H | H | $-\overset{O}{\overset{\|}{C}}-\underset{\underset{CH_3}{\|}}{CH}-C_2H_5$ |
| H | H | H | $-\overset{O}{\overset{\|}{C}}-CH_2-SO_2-CH_3$ |
| H | H | H | $-\overset{O}{\overset{\|}{C}}-NH-CH_3$ |
| H | H | H | $-\overset{O}{\overset{\|}{C}}-NH-C_2H_5$ |

| $R^1$ | $R^2$ | $R^3$ | $R^4$ |
|---|---|---|---|
| H | H | H | $-\overset{O}{\overset{\|}{C}}-N\langle{}^{CH_3}_{C_2H_5}$ |
| H | H | H | 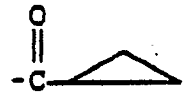 |
| H | H | H | $-\overset{O}{\overset{\|}{C}}-CF_3$ |
| H | H | H | $-\overset{O}{\overset{\|}{C}}-CCl_3$ |
| H | H | H | $-\overset{O}{\overset{\|}{C}}-CClF_2$ |
| H | H | H | $-\overset{O}{\overset{\|}{C}}-CHF_2$ |
| H | H | H | $-\overset{O}{\overset{\|}{C}}-(CH_2)_2-CH_3$ |
| H | H | H | 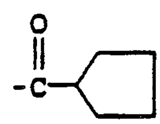 |
| H | H | H | $-\overset{O}{\overset{\|}{C}}-O-(CH_2)_2-CH_3$ |
| H | H | H | $-\overset{O}{\overset{\|}{C}}-CH_2-CH_2-OC_2H_5$ |

| $R^1$ | $R^2$ | $R^3$ | $R^4$ |
|---|---|---|---|
| H | H | H | 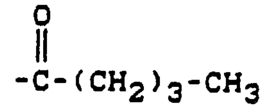 |
| H | H | H | 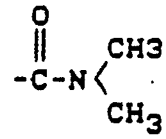 |
| H | H | H | 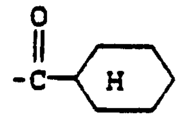 |
| H | H | H | $CH_3$ |
| H | H | H | $C_2H_5$ |
| H | H | $CH_3$ | $CH_3$ |
| H | H | $CH_3$ | $C_2H_5$ |
| H | $NO_2$ | $C_2H_5$ | $C_2H_5$ |
| H | $NO_2$ | H | $CH_3$ |
| H | $NO_2$ | H | $C_2H_5$ |
| H | $NO_2$ | H | $-(CH_2)_2-CH_3$ |
| $CH_3$ | $NO_2$ | H | 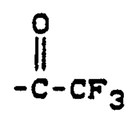 |
| $CH_3$ | $NO_2$ | H | 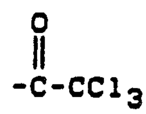 |
| $CH_3$ | $NO_2$ | H | 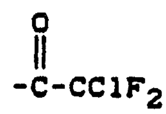 |

| $R^1$ | $R^2$ | $R^3$ | $R^4$ |
|---|---|---|---|
| $CH_3$ | $NO_2$ | H | 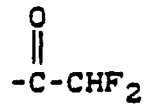 |
| $CH_3$ | $NO_2$ | H | 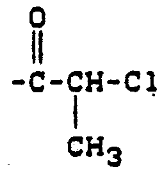 |
| $CH_3$ | $NO_2$ | H | 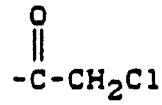 |
| $CH_3$ | $NO_2$ | H | 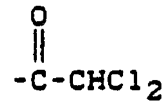 |
| $CH_3$ | $NO_2$ | H | 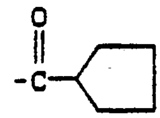 |
| $CH_3$ | $NO_2$ | H | 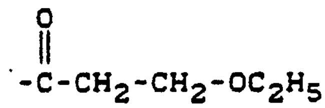 |
| $CH_3$ | $NO_2$ | H | 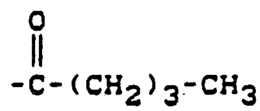 |
| $C_2H_5$ | $NO_2$ | H | 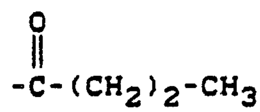 |
| $C_2H_5$ | $NO_2$ | H | 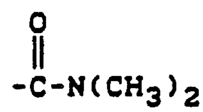 |
| $C_2H_5$ | $NO_2$ | H | 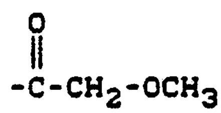 |

| $R^1$ | $R^2$ | $R^3$ | $R^4$ |
|---|---|---|---|
| $CH_3$ | $NO_2$ | H | $-\overset{O}{\overset{\|}{C}}-N(CH_3)_2$ |
| $CH_3$ | $NO_2$ | H | $-\overset{O}{\overset{\|}{C}}-(CH_2)_2-CH_3$ |
| $CH_3$ | $NO_2$ | H | $-\overset{O}{\overset{\|}{C}}-OCH_3$ |
| $CH_3$ | $NO_2$ | H | $-\overset{O}{\overset{\|}{C}}-OC_2H_5$ |
| $CH_3-(CH_2)_2-$ | $NO_2$ | H | $-\overset{O}{\overset{\|}{C}}-OCH_3$ |
| $(CH_3)_3C-$ | $NO_2$ | H | $-\overset{O}{\overset{\|}{C}}-OC_2H_5$ |
| $CH_3$ | $NO_2$ | H | $-\overset{O}{\overset{\|}{C}}-CH(CH_3)_2$ |
| $CH_3$ | $NO_2$ | H | $-\overset{O}{\overset{\|}{C}}-CH_2-OCH_3$ |
| $CH_3$ | $NO_2$ | H | $-\overset{O}{\overset{\|}{C}}-\underset{CH_3}{\underset{\|}{C}}H-OCH_3$ |
| $CH_3$ | $NO_2$ | H | $-\overset{O}{\overset{\|}{C}}-CH_2-OC_2H_5$ |

| $R^1$ | $R^2$ | $R^3$ | $R^4$ |
|---|---|---|---|
| $(CH_3)_2CH-$ | $NO_2$ | H | 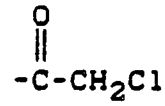 |
| $CH_3-(CH_2)_2-$ | $NO_2$ | H | 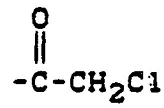 |
| $CH_3$ | $NO_2$ | H | 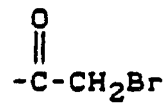 |
| $CH_3$ | $NO_2$ | H | 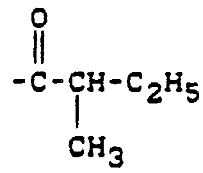 |
| $CH_3$ | $NO_2$ | H | 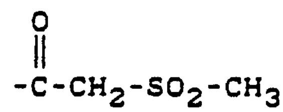 |
| $CH_3$ | $NO_2$ | H | 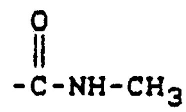 |
| $CH_3$ | $NO_2$ | H | 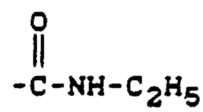 |
| $CH_3$ | $NO_2$ | H | 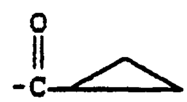 |
| H | $NO_2$ | $CH_3$ | 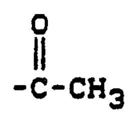 |
| H | $NO_2$ | $C_2H_5$ | 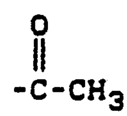 |

| $R^1$ | $R^2$ | $R^3$ | $R^4$ |
|---|---|---|---|
| H | $NO_2$ | $-CH_2-CH=CH_2$ | $-\overset{\overset{O}{\|}}{C}-CH_3$ |
| H | $NO_2$ | $CH_3$ | $-\overset{\overset{O}{\|}}{C}-C_2H_5$ |
| H | $NO_2$ | $C_2H_5$ | $-\overset{\overset{O}{\|}}{C}-C_2H_5$ |
| H | $NO_2$ | $-CH_2-CH=CH-Cl$ | $-\overset{\overset{O}{\|}}{C}-CH_3$ |
| H | $NO_2$ | $-CH_2-C\equiv CH$ | $-\overset{\overset{O}{\|}}{C}-CH_3$ |
| H | $NO_2$ | $-(CH_2)_2-CH_3$ | $-\overset{\overset{O}{\|}}{C}-CH_3$ |
| H | $NO_2$ | H | $-CH_2-CH=CH_2$ |
| H | $NO_2$ | H | $-CH_2-C\equiv CH$ |
| H | $NO_2$ | H | $-CH_2-CH=CH-Cl$ |
| H | $NO_2$ | $CH_3$ | $CH_3$ |
| H | $NO_2$ | $-CH_2-CH=CH_2$ | $-CH_2-CH-CH_2$ |
| H | $NO_2$ | $-CH_2-C\equiv CH$ | $-CH_2-C\equiv CH$ |

Verwendet man beispielsweise 2,4-Dichlor-3,5,6-trifluorphenylhydrazin und Ethoxymethylencyanessigsäureethylester als Ausgangsstoffe, so läßt sich der Reaktionsablauf des erfindungsgemäßen Verfahrens (a-α) durch das folgende Formelschema darstellen:

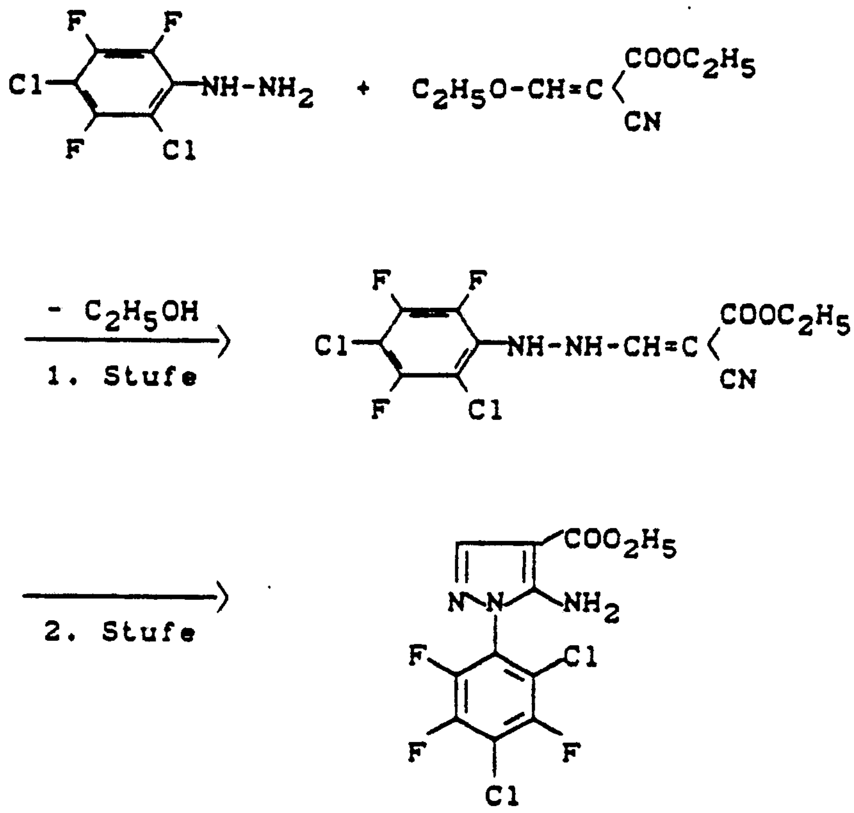


Verwendet man beispielsweise 2,4-Dichlor-3,5,6-trifluorphenylhydrazin und 2-Chloracrylnitril als Ausgangsstoffe, so läßt der Reaktionsablauf des erfindungsgemäßen Verfahrens (a-β) durch das folgende Formelschema darstellen:

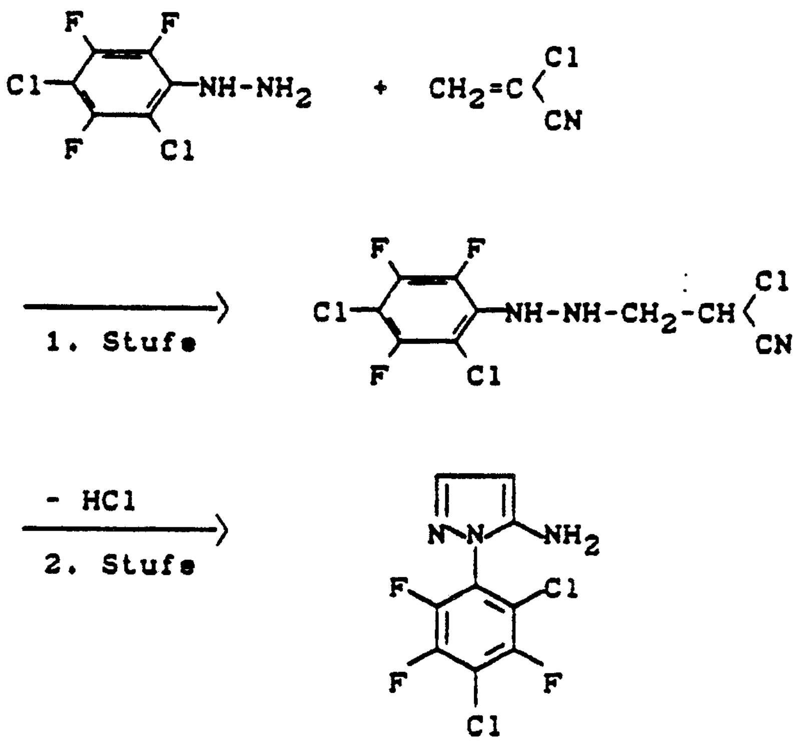


Verwendet man beispielsweise 5-Amino-1-(2,4-dichlor-3,5,6-trifluorphenyl)-pyrazol-4-carbonsäureethyl-

ester als Ausgangsverbindung, so läßt sich der Reaktionsablauf des erfindungsgemäßen Verfahrens (b) durch das folgende Formelschema darstellen:

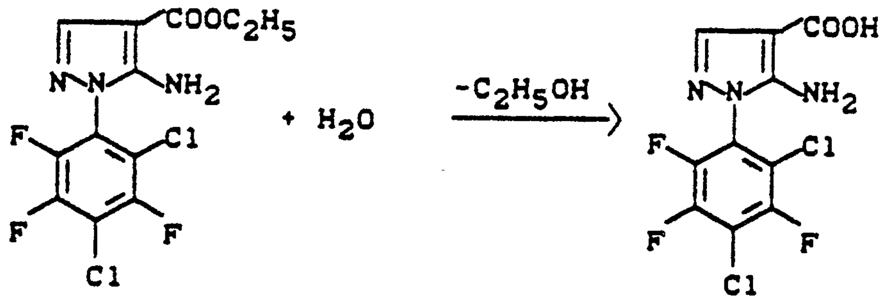


Verwendet man beispielsweise 5-Amino-1-(2,4-dichlor-3,5,6-trifluorphenyl)-pyrazol-4-carbonsäure als Aus gangsverbindung, so läßt sich der Reaktionsablauf des erfindungsgemäßen Verfahrens (c) durch das folgende Formelschema darstellen:

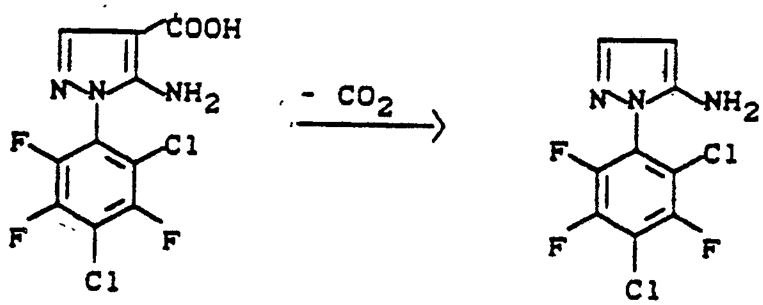


Verwendet man beispielsweise 5-Amino-1-(2,4-dichlor-3,5,6-trifluorphenyl)-pyrazol und Propionylchlorid als Ausgangsstoffe, so läßt sich der Reaktionsablauf des erfindungsgemäßen Verfahrens (d-α) durch das folgende Formelschema darstellen:

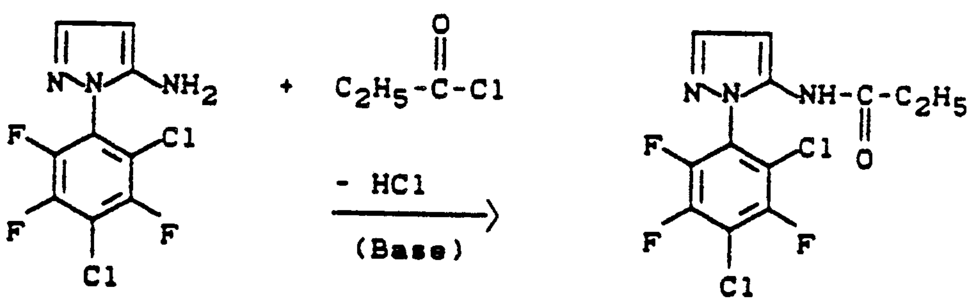


Verwendet man beispielsweise 5-Amino-1-(2,4-dichlor-3,5,6-trifluorphenyl)-pyrazol und Methyliodid als Ausgangsstoffe, so läßt sich der Reaktionsablauf des erfindungsgemäße Verfahrens (d-β) durch das folgende Formelschema darstellen:

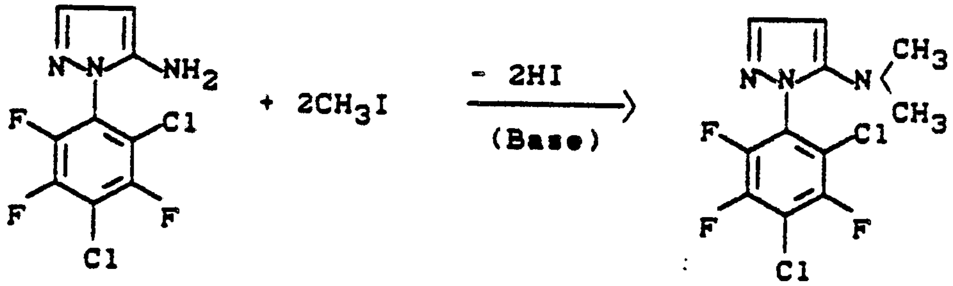

Verwendet man beispielsweise 1-(2,4-Dichlor-3,5,6-trifluorphenyl)-5-propionamidopyrazol und Salpetersäure als Ausgangsstoffe, so läßt sich der Reaktionsablauf des erfindungsgemäßen Verfahrens (e) durch das folgende Formelschema darstellen:

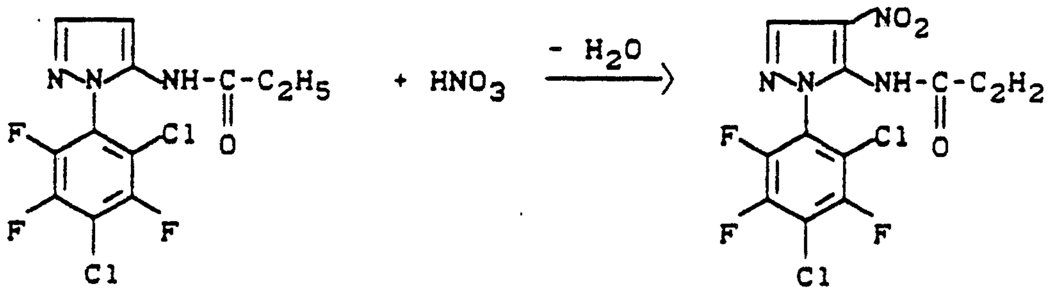


Verwendet man beispielsweise 1-(2,4-Dichlor-3,5,6-trifluorphenyl)-4-nitro-5-(N-methylpropionamido)-pyrazol als Ausgangsverbindung, so läßt sich der Reaktionsablauf des erfindungsgemäßen Verfahrens (f) durch das folgende Formelschema darstellen:

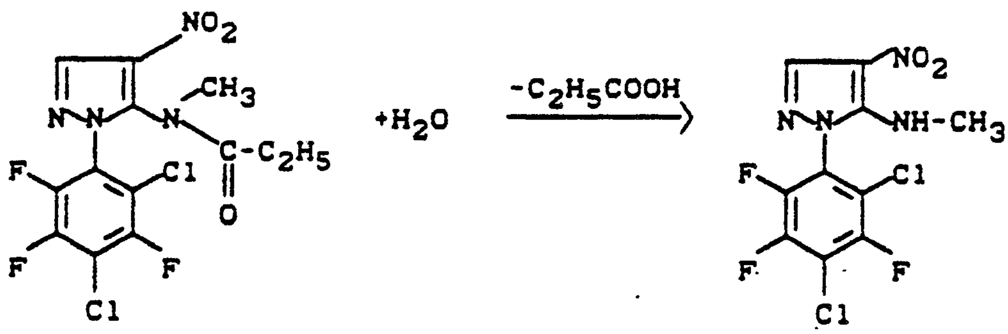


Verwendet man beispielsweise 5-Amino-4-nitro-1-(2,4-dichlor-3,5,6-trifluorphenyl)-pyrazol und Phenylisocyanat als Ausgangsstoffe, so läßt sich der Reaktionsablauf des erfindungsgemäßen Verfahrens (g) durch das folgende Formelschema darstellen:

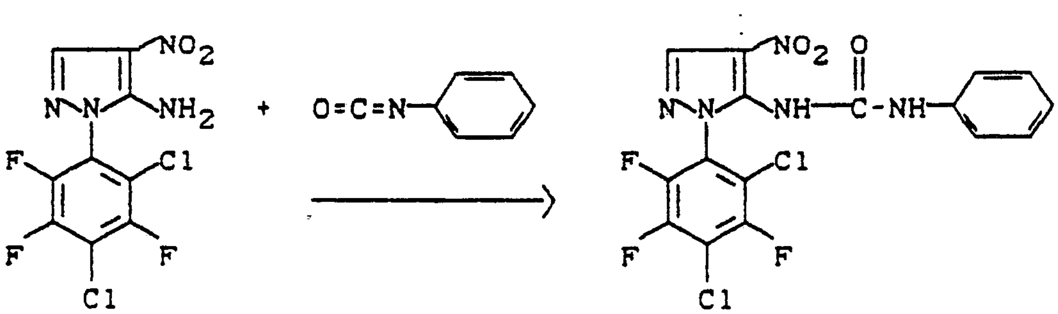

Verwendet man beispielsweise 5-Brom-4-nitro-1-(2,4-dichlor-3,5,6-trifluorphenyl)-pyrazol und Isopropylamin als Ausgangsstoffe, so läßt sich der Reaktionsablauf des erfindungsgemäßen Verfahrens (h) durch das folgende Formelschema darstellen:

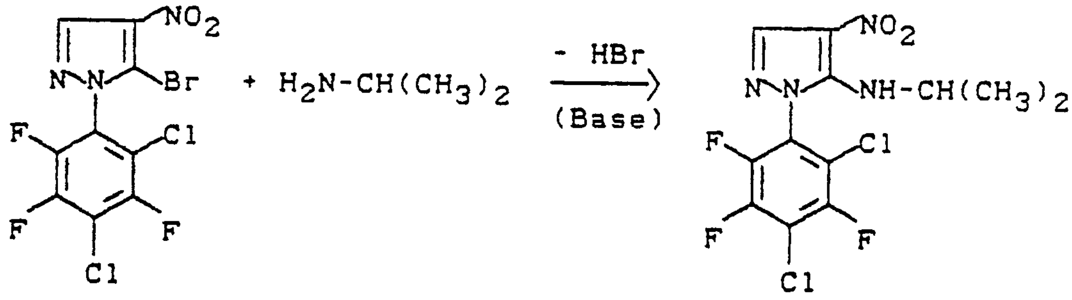


Verwendet man beispielsweise 1-(2,4-Dichlor-3,5,6-trifluorphenyl)-5-phenoxycarbonylaminopyrazol und Methanol als Ausgangsstoffe, so läßt sich der Reaktionsablauf des erfindungsgemäßen Verfahrens (i) durch das folgende Formelschema darstellen:

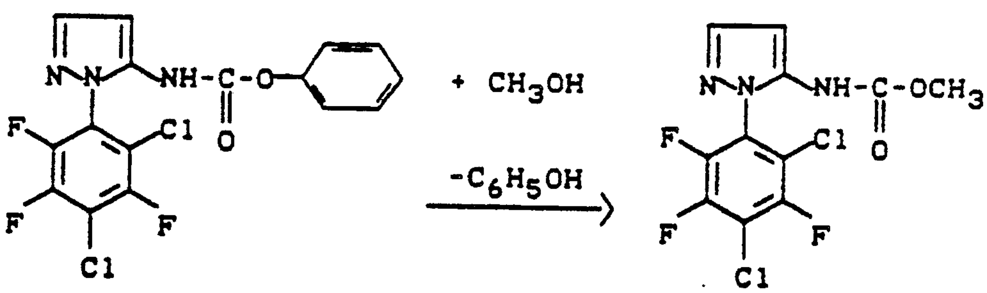


Das zur Durchführung des erfindungsgemäßen Verfahrens (a) als Ausgangsverbindung benötigte 2,4-Dichlor-3,5,6-trifluorphenylhydrazin ist durch die Formel (II) definiert. Das 2,4-Dichlor-3,5,6-trifluorphenylhydrazin der Formel (II) ist bekannt (vgl. z.B. Nippon Kagaku Zasshi [J. chem. Soc. Japan; Pure chem. Sect]-.; 89, 321 [1968] bzw. CA 69: 67 021a).

Die zur Durchführung des erfindungsgemäßen Verfahrens (a-α) weiterhin als Ausgangsstoffe benötigten Acrylnitril-Derivate sind durch die Formel (IIIa) allgemein definiert. In dieser Formel (IIIa) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden. $R^{2-2}$ steht vorzugsweise für geradkettiges oder verzweigtes Alkoxycarbonyl mit 1 bis 5 Kohlenstoffatomen oder für Nitro; insbesondere für Methoxycarbonyl, Ethoxycarbonyl oder Nitro. A steht vorzugsweise für Chlor, Brom, Hydroxy, Methoxy, Ethoxy, Methylamino oder Dimethylamino.

Die zur Durchführung des erfindungsgemäßen Verfahrens (a-β) weiterhin als Ausgangsstoffe benötigten 2-Chlor acrylnitrile sind durch die Formel (IIIb) allgemein definiert. In dieser Formel (IIIb) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Acrylnitril-Derivate der Formeln (IIIa) und (IIIb) sind allgemein bekannt (vgl. DE-OS 31 29 429, DE-OS 32 06 878, EP 34 945; J. Chem. Soc. D. 1255; 1970, Can. J. Chem. 48. 2104-2109 (1970); J. Heterocyclic Chem. 19, 1267-1273 (1982); Can. J. Chem. 51, 1239-1244 (1973)) oder können nach bekannten Verfahren in einfacher analoger Weise erhalten werden.

Die zur Durchführung des erfindungsgemäßen Verfahrens (b) als Ausgangsstoffe benötigten 5-Amino-1-phenyl-pyrazole sind durch die Formel (Iz) allgemein definiert. In dieser Formel (Iz) stehen $R^1$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden. $R^6$ steht vorzugsweise für geradkettiges oder verzweigtes Alkyl mit 1 bis 4 Kohlenstoffatomen, insbesondere für Methyl oder Ethyl.

Die 5-Amino-1-phenyl-pyrazole der Formel (Iz) sind erfindungsgemäße Verbindungen und erhältlich mit Hilfe der erfindungsgemäßen Verfahren (a), (d), (g), (h) oder (i).

Die zur Durchführung des erfindungsgemäßen Verfahrens (c) als Ausgangsstoffe benötigten 5-Amino-1-phenyl-pyrazole sind durch die Formel (Ib) allgemein definiert. In dieser Formel (Ib) stehen $R^1$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-phenyl-pyrazole der Formel (Ib) sind erfindungsgemäße Verbindungen und erhältlich mit Hilfe des erfindungsgemäßen Verfahrens (b).

Die zur Durchführung der erfindungsgemäßen Verfahren (d) und (g) als Ausgangsstoffe benötigten 5-Amino-1-phenylpyrazole sind durch die Formel (Iy) allgemein definiert. In dieser Formel (Iy) stehen $R^1$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden. $R^{2-1}$ steht vorzugsweise für Wasserstoff, für geradkettiges oder verzweigtes Alkoxycarbonyl mit 1 bis 5 Kohlenstoffatomen oder für Nitro, insbesondere für Wasserstoff, Methoxycarbonyl, Ethoxycarbonyl oder Nitro.

Die 5-Amino-1-phenylpyrazole der Formel (Iy) sind erfindungsgemäße Verbindungen.

5-Amino-1-phenylpyrazole der Formel (Iy), bei welchen $R^3$ für Wasserstoff steht, sind erhältlich mit Hilfe der erfindungsgemäßen Verfahren (a), (c) oder (e). 5-Amino-1-phenylpyrazole der Formel (Iy), in welchen $R^3$ verschieden von Wasserstoff ist, sind erhältlich mit Hilfe der erfindungsgemäßen Verfahren (c), (e), (f), (h) oder (i).

Außerdem können 5-Amino-1-phenylpyrazole der Formel (Iy), welche beispielsweise nach dem erfindungsgemäßen Verfahren (d-α) oder (g) hergestellt werden, im erfindungsgemäßen Verfahren (d-β) als Ausgangsstoffe eingesetzt werden.

Setzt man die mit Hilfe der erfindungsgemäßen Verfahren (d-α), (d-β) oder (g) erhaltenen mono-alkylierten oder mono-acylierten Verbindungen erneut nach einem dieser Verfahren um, erhält man die entsprechenden disubstituierten Verbindungen.

Die zur Durchführung des erfindungsgemäßen Verfahrens (d-α) weiterhin als Ausgangsstoffe benötigten Acylierungsmittel sind durch die Formel (V) allgemein definiert. In dieser Formel (V) stehen $R^5$ und X vorzugsweise für diejenigen Reste, die bereits im Zusammenhang mit der Beschreibung der erfindungsgemäßen Stoffe der Formel (I) als bevorzugt für diese Substituenten genannt wurden.

$A^1$ steht vorzugsweise für Halogen, insbesondere für Chlor oder Brom oder für einen Rest $R^5$-CO-O-, wobei $R^5$ die oben genannte Bedeutung hat.

Die zur Durchführung des erfindungsgemäßen Verfahrens (d-β) weiterhin als Ausgangsstoffe benötigten Alkylierungsmittel sind durch die Formel (VI) allgemein definiert. In dieser Formel (VI) steht $R^{4-2}$ vorzugsweise für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen.

$R^{4-2}$ steht besonders bevorzugt für Methyl, Ethyl, n-oder i-Propyl, n-, i-, s- oder t-Butyl, für Allyl, n-oder i-Butenyl, Propargyl, n- oder i-Butinyl, für Chlorethyl, Trifluorethyl, Bromethyl, Chlorallyl, Dichlorallyl oder Brompropargyl.

$R^{4-2}$ steht insbesondere für Methyl, Ethyl, n- oder i-Propyl, n-, i-, s- oder t-Butyl, für Allyl, Chlorallyl oder Propargyl.

$A^2$ steht vorzugsweise für Chlor, Brom oder Iod, für p-Toluolsulfonyloxy, für Methoxysulfonyloxy oder für Ethoxysulfonyloxy.

Die zur Durchführung des erfindungsgemäßen Verfahrens (g) weiterhin als Ausgangsstoffe benötigten Iso(thio)cyanate sind durch die Formel (VII) allgemein definiert. In dieser Formel (VII) steht X vorzugsweise für diejenigen Reste, die bereits im Zusammenhang mit der Be schreibung der erfindungsgemäßen Stoffe der Formel (I) als bevorzugt für diesen Substituenten genannt wurden.

$R^7$ steht vorzugsweise für geradkettiges oder verzweigtes Alkyl mit 1 bis 4 Kohlenstoffatomen, insbesondere für Methyl oder Ethyl oder für gegebenenfalls einfach oder mehrfach, insbesondere ein- bis dreifach, gleich oder verschieden substituiertes Phenyl, wobei als Substituenten infrage kommen: Halogen sowie jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen, insbesondere kommen als Substituenten Fluor, Chlor, Methyl, Methoxy oder Trifluormethyl infrage.

Die Acylierungsmittel der Formel (V), die Alkylierungsmittel der Formel (VI) und die Iso(thio)cyanate der Formel (VII) sind allgemein bekannte Verbindungen der organischen Chemie.

Die zur Durchführung des erfindungsgemäßen Verfahrens (e) als Ausgangsstoffe benötigten 5-Amino-1-phenyl-pyrazole sind durch die Formel (Ic) allgemein definiert. In dieser Formel (Ic) stehen $R^1$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-phenylpyrazole der Formel (Ic) sind erfindungsgemäße Verbindungen und erhältlich mit Hilfe der erfindungsgemäßen Verfahren (a), (c), (d), (f), (g), (h) oder (i).

Die zur Durchführung des erfindungsgemäßen Verfahrens (f) als Ausgangsverbindungen benötigten 5-Amino-1-phenylpyrazole sind durch die Formel (Ix) allgemein definiert.

In dieser Formel (Ix) stehen $R^1$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden. $R^{2-3}$ steht vorzugsweise für Nitro oder Wasserstoff, $R^{4-3}$ steht vorzugsweise für Wasserstoff, für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen.

$R^{4-3}$ steht besonders bevorzugt für Wasserstoff, Methyl, Ethyl, n- oder i-Propyl, n-, i-, s- oder t-Butyl, für Allyl, n- oder i-Butenyl, Propargyl, n- oder i-Butinyl, für Chlorethyl, Trifluorethyl, Bromethyl, Chlorallyl, Dichlorallyl oder Brompropargyl.

$R^{4-3}$ steht insbesondere für Wasserstoff, Methyl, Ethyl, n- oder i-Propyl, n-, i-, s- oder t-Butyl, für Allyl, Chlorallyl oder Propargyl.

Die 5-Amino-1-phenylpyrazole der Formel (Ix) sind erfindungsgemäße Verbindungen und erhältlich mit Hilfe der erfindungsgemäßen Verfahren (d) oder (e).

Die zur Durchführung des erfindungsgemäßen Verfahrens (h) als Ausgangsstoffe benötigten 5-Halogenpyrazole sind durch die Formel (VIII) allgemein definiert. In dieser Formel (VIII) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

$R^{2-1}$ steht vorzugsweise für Wasserstoff, für geradkettiges oder verzweigtes Alkoxycarbonyl mit 1 bis 5 Kohlenstoffatomen oder für Nitro, insbesondere für Wasserstoff, Methoxycarbonyl, Ethoxycarbonyl oder Nitro.

Hal steht vorzugsweise für Chlor, Brom oder Iod, insbesondere für Chlor oder Brom.

Die 5-Halogenpyrazole der Formel (VIII) sind noch nicht bekannt und ebenfalls Gegenstand der vorliegenden Erfindung.

Man erhält sie in Analogie zu bekannten Verfahren (vgl. z.B. DE-OS 35 01 323), wenn man Alkoxymethylenmalonester der Formel (XI),

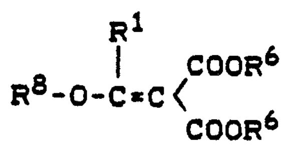

(XI)

in welcher

$R^1$ die oben angegebene Bedeutung hat und

$R^6$ und $R^8$ unabhängig voneinander jeweils für Alkyl, insbesondere für Methyl oder Ethyl stehen,

mit 2,4-Dichlor-3,5,6-trifluorphenylhydrazin der Formel (II)

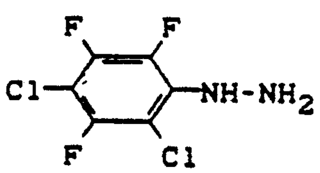

(II)

zunächst in einer ersten Stufe gegebenenfalls in Gegenwart eines Verdünnungsmittels wie beispielsweise Methanol oder Ethanol bei Temperaturen zwischen +10 °C und +80 °C umsetzt, zu den so erhältlichen Pyrazolcarbonsäureester der Formel (XII),

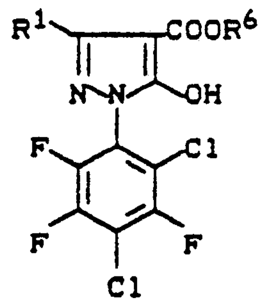

(XII)

in welcher
$R^1$ und $R^6$ die oben angegebene Bedeutung haben,
diese gegebenenfalls in einer zwischengeschalteten 2. Stufe gegebenenfalls in Gegenwart eines Verdünnungsmittels wie beispielsweise Methanol und gegebenenfalls in Gegenwart einer Base wie beispielsweise Natriumhydroxid bei Temperaturen zwischen +30 °C und +70 °C verseift und decarboxyliert zu Pyrazolinonen der Formel (XIII),

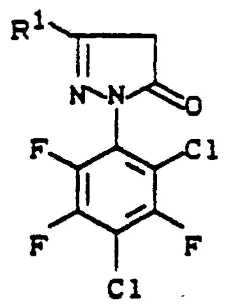

(XIII)

in welcher
$R^1$ die oben angegebene Bedeutung hat,
und entweder die Pyrazolcarbonsäureester der Formel (XII) oder die Pyrazolinone der Formel (XIII) in einer 3. Stufe mit Halogenierungsmitteln, wie beispielsweise Phosphoroxychlorid oder Phosphoroxybromid, nach üblichen bekannten Verfahren (vgl. z.B. Ber. dtsch. chem. Ges. 28, 35 [1895] oder Liebigs Ann. Chem. 373, 129 [1910[) umsetzt, und gegebenenfalls in einer 4. Stufe die so erhältlichen 5-Halogen-pyrazole der Formel (VIIIa),

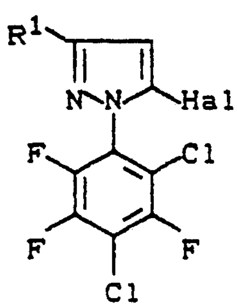

(VIIIa)

in welcher
$R^1$ und Hal die oben angegebene Bedeutung haben,
mit Nitrierungsmitteln wie beispielsweise Salpetersäure gegebenenfalls in Gegenwart eines Verdünnungsmittels wie beispielsweise Eisessig und gegebenenfalls in Gegenwart eines Reaktionshilfsmittels oder Katalysators wie beispielsweise Acetanhydrid oder Schwefelsäure in Analogie zur Durchführung des erfindungsgemäßen Verfahrens (e) in 4-Stellung des Pyrazolringes nitriert.

Die bei der Umsetzung von Alkoxymethylenmalonestern der Formel (XI) mit 2,4-Dichlor-3,5,6-trifluorphenylhydrazin der Formel (II) auftretenden Zwischenprodukte der Formel (XIV),

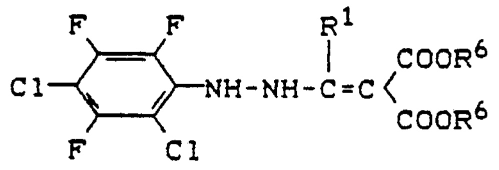

(XIV)

in welcher
$R^1$ und $R^6$ die oben angegebene Bedeutung haben,
können gegebenenfalls auch isoliert und in einer separaten Reakationsstufe cyclisiert werden.

Die Cyclisierung zu den Pyrazolcarbonsäureestern der Formel (XII) und deren anschließende Verseifung und Decarboxylierung können gegebenenfalls in einer Reaktionsstufe als "Eintopfverfahren" durchgeführt werden (vgl. z.B. Liebigs Ann. Chem. 373, 142 [1910]).

Die Alkoxymethylenmalonester der Formel (XI) sind allgemein bekannte Verbindungen der organischen Chemie.

Die zur Durchführung des erfindungsgemäßen Verfahrens (h) weiterhin als Ausgangsstoffe benötigten Amine sind durch die Formel (IX) allgemein definiert. In dieser Formel (IX) steht $R^{3-1}$ vorzugsweise für Wasserstoff oder für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen.

$R^{3-1}$ steht besonders bevorzugt für Wasserstoff, Methyl, Ethyl, n- oder i-Propyl, n-, i-, s- oder t-Butyl, für Allyl, n- oder i-Butenyl, Propargyl, n- oder i-Butinyl, für Chlorethyl, Trifluorethyl, Bromethyl, Chlorallyl, Dichlorallyl oder Brompropargyl.

$R^{4-2}$ steht vorzugsweise für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen.

$R^{4-2}$ steht besonders bevorzugt für Methyl, Ethyl, n-oder i-Propyl, n-, i-, s- oder t-Butyl, für Allyl, n-oder i-Butenyl, Propargyl, n- oder i-Butinyl, für Chlorethyl, Trifluorethyl, Bromethyl, Chlorallyl, Dichlorallyl oder Brompropargyl.

Die Amine der Formel (IX) sind ebenfalls allgemein bekannte Verbindungen der organischen Chemie.

Die zur Durchführung des erfindungsgemäßen Verfahrens (i) als Ausgangsstoffe benötigten (Bis)-Carbamate sind durch die Formel (Iw) allgemein definiert. In dieser Formel (Iw) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden, $R^{2-1}$ steht vorzugsweise für Wasserstoff, für geradkettiges oder verzweigtes Alkoxycarbonyl mit 1 bis 5 Kohlenstoffatomen oder für Nitro, insbesondere für Wasserstoff, Methoxycarbonyl, Ethoxycarbonyl oder Nitro. $R^{3-2}$ steht für einen Rest -CO-O-Ar oder für Wasserstoff, wobei Ar vorzugsweise für Phenyl steht.

Die (Bis)Carbamate der Formel (Iw) sind erfindungsgemäße Verbindungen und erhältlich mit Hilfe der erfindungsgemäßen Verfahren (d) oder (e).

Die zur Durchführung des erfindungsgemäßen Verfahrens (i) weiterhin als Ausgangsstoffe benötigten Alkohole, Amine oder Thiole sind durch die Formel (X) allgemein definiert. In dieser Formel (X) steht $R^{5-1}$ vorzugsweise für jeweils geradkettiges oder verzweigtes Alkoxy, Alkylthio, Alkylamino oder Dialkylamino mit jeweils 1 bis 4 Kohlenstoffatomen in den einzelnen Alkylteilen, für jeweils einfach oder mehrfach, gleich oder verschieden substituiertes Phenoxy, Phenylthio oder Phenylamino, wobei als Phenylsubstituenten jeweils in Frage kommen: Halogen, jeweils geradkettiges oder verzweigtes Alkyl oder Alkoxy mit jeweils 1 bis 4 Kohlenstoffatomen, für geradkettiges oder verzweigtes Halogenalkyl mit 1 bis 4 Koh lenstoffatomen und 1 bis 9 gleichen oder verschiedenen Halogenatomen, insbesondere Methyl, Methoxy, Chlor oder Trifluormethyl, $R^{5-1}$ steht insbesondere für Methoxy, Ethoxy, Methylthio, Phenylthio oder Dimethylamino.

Die Alkohole, Amine oder Thiole der Formel (X) sind allgemein bekannte Verbindungen der organischen Chemie.

Als Verdünnungsmittel zur Durchführung der Herstellungsverfahren (a-α) und (a-β) kommen sowohl für die 1. als auch für die 2. Reaktionsstufe inerte organische Lösungsmittel in Frage. Vorzugsweise verwendet man Alkohole, wie Methanol, Ethanol, Propanol, Butanol, Ethylenglykol oder Ethylenglykolmonomethyl- oder -monoethylether.

Als Reaktionshilfsmittel zur Durchführung der 1. Stufe der Herstellungsverfahren (a-α) und (a-β) kommen organische oder anorganische Säuren in Frage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe der Herstellungsverfahren (a-α) und (a-β) in gewissen Bereichen variiert werden. Im allgemeinen arbeitet man zwischen -30 °C und +50 °C, vorzugsweise zwischen -20 °C und +20 °C.

Als Reaktionshilfsmittel zur Durchführung der 2. Stufe der Herstellungsverfahren (a-α) und (a-β) kommen alle üblicherweise verwendbaren anorganischen und organischen Basen in Frage. Vorzugsweise verwendet man Alkalimetallcarbonate oder-Hydrogencarbonate, wie Natriumcarbonat, Kaliumcarbonat oder Kaliumhydrogencarbonat.

Die Reaktionstemperaturen können bei der Durchführung der 2. Stufe der Herstellungsverfahren (a-α) und (a-β) ebenso wie bei der einstufigen Reaktionsführung in einem größeren Bereich variiert werden. Im allgemeinen arbeitet man zwischen 0 °C und 200 °C, vorzugsweise zwischen +50 °C und +150 °C.

Zur Durchführung der Herstellungsverfahren (a-α) und (a-β) setzt man sowohl bei der einstufigen als auch bei der zweistufigen Reaktionsführung pro Mol an 2,4-Dichlor-3,5,6-trifluorphenylhydrazin der Formel (II) im allgemeinen 1,0 bis 3,0 Mol, vorzugsweise 1,0 bis 1,5 Mol an Acrylnitril-Derivat der Formel (IIIa) oder (IIIb) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an Säurebindemittel ein.

Die erfindungsgemäßen Verfahren (a-α) und (a-β) können auch direkt in einem Reaktionsschritt ohne Isolierung der Zwischenprodukte der Formel (IV) durchgeführt werden.

Die Aufarbeitung und Isolierung der Reaktionsprodukte der Formel (Ia) erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Als Verdünnungsmittel zur Durchführung des Herstellungsverfahrens (b) kommen anorganische oder organische Lösungsmittel in Frage. Vorzugsweise verwendet man polare Lösungsmittel, insbesondere Alkohole, wie beispielsweise Methanol, Ethanol oder Propanol, oder deren Gemische mit Wasser.

Als Reaktionshilfsmittel zur Durchführung des Herstellungsverfahrens (b) kommen alle üblicherweise für derartige Esterverseifungen verwendbaren Katalysatoren in Frage. Vorzugsweise verwendet man Basen, wie beispielsweise Natriumhydroxid, Natriumalkoholat oder Natriumcarbonat oder Säuren, wie beispielsweise Chlorwasserstoffsäure, Bromwasserstoffsäure oder Schwefelsäure.

Die Reaktionstemperaturen können bei der Durchführung des erfindungsgemäßen Verfahrens (b) in einem größeren Bereich variiert werden. Im allgemeinen arbeitet man bei Temperaturen zwischen 20 °C und 150 °C, vorzugsweise bei Temperaturen zwischen 50 °C und 100 °C.

Zur Durchführung des Herstellungsverfahrens (b) setzt man pro Mol an 5-Amino-1-phenyl-pyrazol der Formel (Iz) im allgemeinen 1,0 bis 15,0 Mol, vorzugsweise 1,0 bis 2,5 Mol an saurem oder basischem Reaktionshilfsmittel ein und erwärmt für mehrere Stunden auf die erforderliche Reaktionstemperatur. Die Aufarbeitung, Isolierung und Reinigung der Reaktionsprodukte der Formel (Ib) erfolgt nach üblichen Verfahren.

Als Verdünnungsmittel zur Durchführung des Herstellungs verfahrens (c) kommen ebenfalls anorganische oder organische, vorzugsweise polare Lösungsmittel in Frage. Insbesondere sind Alkohole, wie beispielsweise Methanol, Ethanol oder Propanol, oder deren Gemische mit Wasser geeignet.

Als Katalysatoren zur Durchführung des Herstellungsverfahrens (c) kommen vorzugsweise Säuren, insbesondere anorganische Mineralsäuren wie Chlorwasserstoffsäure, Bromwasserstoffsäure oder Schwefelsäure in Frage.

Die Reaktionstemperaturen können bei der Durchführung des Herstellungsverfahrens (c) in einem größeren Bereich variiert werden. Im allgemeinen arbeitet man zwischen +50 °C und +200 °C, vorzugsweise zwischen +70 °C und +130 °C.

Bei der Durchführung des erfindungsgemäßen Verfahrens (c) setzt man pro Mol an 5-Amino-1-phenylpyrazol der Formel (Ib) im allgemeinen 1,0 bis 30,0 Mol, vorzugsweise 1,0 bis 15,0 Mol an Katalysatorsäure ein und erwärmt für mehrere Stunden auf die erforderliche Temperatur. Die Aufarbeitung, Isolierung und Reinigung der Reaktionsprodukte der Formel (Ic) erfolgt nach allgemein üblichen Verfahren.

Bei Verwendung eines sauren Katalysators ist es auch möglich die erfindungsgemäßen Verfahren (b) (Esterverseifung) und (c) (Decarboxylierung) in einem Reaktionsschritt als Eintopfverfahren durchzuführen. Auch in diesem Fall erfolgt die Reaktionsdurchführung, Aufarbeitung und Isolierung der Reaktionsprodukte nach allgemein üblichen Methoden (vergl. auch Herstellungsbeispiele).

Zur Durchführung des erfindungsgemäßen Verfahrens (d) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan,

Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril oder Amide, wie Dimethylformamid, Dimethylacetamid, N-Methylpyrrolidon oder Hexamethylphosphorsäuretriamid. Verwendet man Acylierungsmittel der Formel (V) oder Alkylierungsmittel der Formel (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (d) kommen alle üblicherweise verwendbaren anorganischen und anorganischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethyl amin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (d) in einem größeren Bereich variiert werden. Im allgemeinen arbeitet man zwischen -20 °C und +150 °C, vorzugsweise zwischen 0 °C und +100 °C.

Zur Durchführung des Herstellungsverfahrens (d) setzt man pro Mol 5-Amino-1-phenyl-pyrazol der Formel (Iy) im allgemeinen 1,0 bis 20,0 Mol, vorzugsweise 1,0 bis 15,0 Mol an Acylierungsmittel der Formel (V) oder an Alkylierungsmittel der Formel (VI) und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Id) erfolgt in allgemein üblicher Art und Weise.

Als Nitrierungsmittel zu Durchführung des erfindungsgemäßen Verfahrens (e) kommen alle üblichen Nitrierungsmittel in Frage. Vorzugsweise verwendet man konzentrierte Salpetersäure oder Nitriersäure.

Als Verdünnungsmittel zur Durchführung des erfindungsgemäßen Verfahrens (e) kommen alle üblicherweise für derartige Nitrierungsreaktionen verwendbaren Lösungsmittel in Frage. Vorzugsweise verwendet man die als Reagenzien in Frage kommenden Säuren oder Gemische, wie beispielsweise Schwefelsäure, Salpetersäure oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des Herstellungsverfahrens (e) kommen ebenfalls die für derartige Reaktionen üblichen Katalysatoren in Frage; vorzugsweise verwendet man saure Katalysatoren wie beispielsweise Schwefelsäure oder Acetanhydrid.

Die Reaktionstemperaturen können bei der Durchführung des Herstellungsverfahrens (e) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (e) setzt man pro Mol 5-Amino-1-phenylpyrazol der Formel (Ic), im allgemeinen 1,0 bis 10,0 Mol, vorzugsweise 1,0 bis 5,0 Mol an Nitrierungsmittel und gegebenenfalls 0,1 bis 10 Mol Reaktionshilfsmittel ein. Die Reaktionsführung, Aufarbeitung und Isolierung der Reaktionsprodukte der Formel (Ie) erfolgt in allgemein üblicher Art und Weise.

Als Verdünnungsmittel zur Durchführung des erfindungsgemäßen Verfahrens (f) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Reaktionshilfsmittel zur Durchführung des Herstellungsverfahrens (f) kommen vorzugsweise Säuren, insbesondere Chlorwasserstoffsäure oder Schwefelsäure in Frage.

Die Reaktionstemperaturen können bei der Durchführung des Herstellungsverfahrens (f) in einem größeren Bereich variiert werden. Im allgemeinen arbeitet man zwischen +20 °C und +150 °C, vorzugsweise zwischen +50 °C und +120 °C.

Zur Durchführung des Herstellungsverfahrens (f) setzt man pro Mol 5-Amino-1-phenylpyrazol der Formel (Ix) im allgemeinen 1,0 bis 20,0 Mol, vorzugsweise 1,0 bis 10,0 Mol an Reaktionshilfsmittel ein und erwärmt für mehrere Stunden auf die erforderliche Reaktionstemperatur. Die Aufarbeitung, Isolierung und Reinigung der Reaktionsprodukte der Formel (If) erfolgt nach üblichen Methoden.

Zur Durchführung des erfindungsgemäßen Verfahrens (g) kommen als Verdünnungsmittel inerte organische Lösungsmittel in Frage. Vorzugsweise verwendet man die bei Verfahren (d) genannten Verdünnungsmittel. Verwendet man Iso(thio)cyanate der Formel (VII) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (g) kommen tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU), in Frage.

Die Reaktionstemperaturen können bei der Durchführung des Verfahrens (g) in einem größeren Bereich variiert werden. Im allgemeinen arbeitet man bei Temperaturen zwischen -20 °C und +150 °C, vorzugsweise zwischen 0 °C und +100 °C.

Zur Durchführung des Herstellungsverfahrens (g) setzt man pro Mol 5-Amino-1-phenylpyrazol der Formel (Iy) im allgemeinen 1,0 bis 20,0 Mol, vorzugsweise 1,0 bis 15,0 Mol an Iso(thio)cyanat der Formel (VII) und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Ig) erfolgt in allgemein üblicher Art und Weise.

Als Verdünnungsmittel zur Durchführung des erfindungsgemäßen Verfahrens (h) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (h) kann gegebenenfalls in Gegenwart eines geeigneten Reaktionshilfsmittels durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (IX) gleichzeitig als Säurebindemittel zu verwenden.

Die Reaktionstemperaturen können bei der Durchführung des erfindungsgemäßen Verfahrens (h) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (h) setzt man pro Mol an 5-Halogenpyrazol der Formel (VIII) im allgemeinen 1,0 bis 10,0 Mol, vorzugsweise 1,0 bis 5,0 Mol an Amin der Formel (IX) ein. Die Reaktionsdurchführung, Aufarbeitung und Isolierung der Reaktionsprodukte der Formel (Ih) erfolgt nach allgemein üblichen Verfahren.

Als Verdünnungsmittel zur Durchführung der erfindungsgemäßen Verfahren (i) kommen inerte organische Lösungsmittel in Frage. Vorzugsweise verwendet man aliphatische, oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff,Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid, oder Alkohole wie Methanol, Ethanol oder Isopropanol.

Es ist jedoch auch möglich, die als Reaktionskomponenten verwendeten Alkohole, Amine oder Thiole der Formel (X) in entsprechendem Überschuß gleichzeitig als Verdünnungsmittel einzusetzen.

Das erfindungsgemäße Verfahren (i) kann gegebenenfalls in Gegenwart eines geeigneten Reaktionshilfsmittels durchgeführt werden. Als solche kommen alle üblichen anorganischen oder organischen Basen in Frage. Vorzugsweise verwendet man die bei Verfahren (h) genannten Basen.

Die Reaktionstemperaturen können bei dem erfindungsgemäßen Verfahren (i) ebenfalls in einem größeren Bereich variiert werden. Im allgemeinen arbeitet man bei Temperaturen zwischen 0 °C und +200 °C, vorzugsweise zwischen +20 °C und +150 °C.

Zur Durchführung des erfindungsgemäßen Verfahrens (i) setzt man pro Mol (Bis)Carbamat der Formel (Iw) im allgemeinen 1 bis 20 Mol, vorzugsweise 1 bis 10 Mol an Alkohol, Amin oder Thiol der Formel (X) und gegebenenfalls 0,1 bis 2 Mol, vorzugsweise 0,1 bis 1 Mol Reaktionshilfsmittel ein und erwärmt für mehrere Stunden auf die erforderliche Temperatur. Die Aufarbeitung und Isolierung der Reaktionsprodukte der Formel (Ii) erfolgt nach üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selek tive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotina, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung dikotyler Unkräuter in monokotylen Kulturen wie beispielsweise Weizen oder Gerste einsetzen.

Auch die Zwischenprodukte der Formel (IV) und die Vorprodukte der Formel (VIII) besitzen eine herbizide Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine,z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester,

Polyoxyethylen-Fettalkohol- Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablauge und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage.
Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); Methyl-6,6-dimethyl-2,4-dioxo-3-[1-(2-propenyloxyamino)-butyliden]-cyclohexancarbonsäure (ALLOXYDIM); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2,6-Dichlorbenzonitril (DICHLOBENIL); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chloro-2-benzoxazo lyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); 4-(4-Chlor-2-methyl)-phenoxybuttersäure (MCPB); (4-Chlor-2-methylphenoxy)-propionsäure (MCPP); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 3,5,6-Trichlor-2-pyridyloxyessigsäure (TRICLOPYR) sind gegebenenfalls von Vorteil. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weiteres Verdünnen bereite ten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung der erfindungsgemäßen Verbindungen als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

<u>Herstellungsbeispiele</u>

Beispiel 1

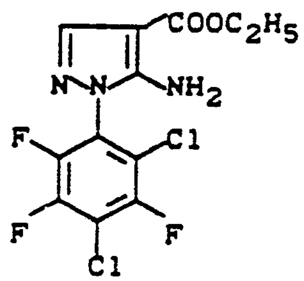

(Verfahren a-α)

60 g (0.296 Mol) 2,4-Dichlor-3,5,6-trifluorphenylhydrazin und 27,8 g (0.161 Mol) Ethoxymethylencyanessigsäureethylester werden in 120 ml Ethoxyethanol gelöst und 10 Stunden unter Rückfluß erwärmt. Das Lösungsmittel wird im Vakuum abdestilliert und der Rückstand in 100 ml Diethylether suspendiert, filtriert und getrocknet.

Man erhält 100 g (95,4 % der Theorie) 5-Amino-4-ethoxycarbonyl-1-(2,4-dichlor-3,5,6-trifluorphenyl)-pyrazol vom Schmelzpunkt 85 °C - 87 °C.

Beispiel 2

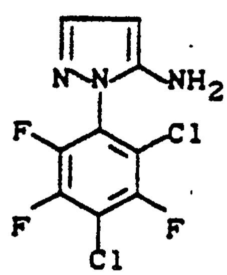

(Verfahren b + c)

100 g (0.282 Mol) 5-Amino-4-ethoxycarbonyl-1-(2,4-Dichlor-3,5,6-trifluorphenyl)-pyrazol werden in 200 ml 50 %iger wäßriger Schwefelsäure suspendiert und innerhalb von 2 Stunden auf 120 °C erwärmt; dabei werden die leichter flüchtigen Anteile über eine Brücke abdestilliert. Man hält die Temperatur weitere 3 Stunden bei 115 °C - 120 °C, kühlt ab, verdünnt mit 400 ml Wasser, stellt mit verdünnter wäßriger Natronlauge einen pH-Wert von 2 ein und saugt den entstandenen Niederschlag ab. Er wird mit Wasser neutral gewaschen und im Vakuum getrocknet.

Man erhält 60,0 g (76 % der Theorie) 5-Amino-1-(2,4-dichlor-3,5,6-trifluorphenyl)-pyrazol vom Schmelzpunkt 78 °C - 82 °C.

Beispiel 3

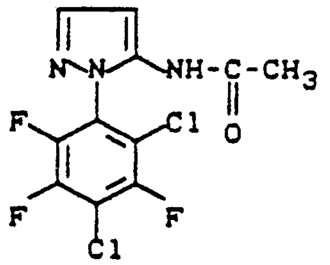

(Verfahren d-α)

Zu 56,4 g (0.20 Mol) 5-Amino-1-(2,4-dichlor-3,5,6-trifluorphenyl)-pyrazol in 170 ml Eisessig gibt man 25 ml (0.26 Mol) Essigsäureanhydrid, wobei die Reaktionstemperatur von 20 °C auf ca. 35 °C ansteigt. Man rührt 16 Stunden bei Raumtemperatur und trägt danach in 800 ml Wasser aus. Der ausgefallene kristalline Feststoff wird abgesaugt, mit Wasser essigsäurefrei gewaschen und im Vakuum getrocknet.

Man erhält 54,4 g (84 % der Theorie) 5-Acetamido-2-(2,4-dichlor-3,5,6-trifluorphenyl)-pyrazol vom Schmelzpunkt 170 °C - 172 °C.

Beispiel 4

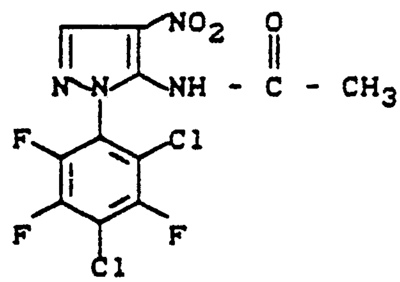

(Verfahren e)

Zu 32,4 g (0.10 Mol) 5-Acetamido-1-(2,4-dichlor-3,5,6-trifluorphenyl)-pyrazol in 85 ml Eisessig gibt man bei ca. 15 °C nacheinander 12,5 ml (0.13 Mol) Essigsäureanhydrid und 4,5 ml (0.105 Mol) 98 %ige Salpetersäure. Man läßt die Temperatur langsam auf 25 °C steigen und rührt 4 Stunden nach. Anschließend wird die Reaktionslösung in 250 ml Wasser ausgetragen. Der Niederschlag wird abfiltriert, neutral gewaschen und im Vakuum getrocknet.

Man erhält 35,2 g (95,5 % der Theorie) 5-Acetamido-4-nitro-1-(2,4-dichlor-3,5,6-trifluorphenyl)-pyrazol vom Schmelzpunkt 137 °C - 138 °C.

Beispiel 5

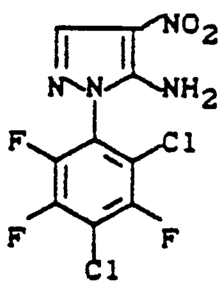

(Verfahren f)

18,5 g (0.05 Mol) 5-Acetamido-4-nitro-1-(2,4-dichlor-3,5,6-trifluorphenyl)-pyrazol werden in einer Mischung aus 40 ml 37 %iger Salzsäure und 70 ml Ethanol 7 Stunden unter Rückfluß erhitzt. Die Reaktionsmischung wird im Vakuum vom Ethanol befreit. Den Rückstand versetzt man mit 35 ml Wasser und neutralisiert mit verdünnter Natronlauge. Der Niederschlag wird abgesaugt, mit Wasser gewaschen und im Vakuum getrocknet.

Man erhält 15,0 g (92 % der Theorie) 5-Amino-4-nitro-1-(2,4-dichlor-3,5,6-trifluorphenyl)-pyrazol vom Schmelzpunkt 170 °C - 173 °C.

Beispiel 5 (alternative Herstellung)

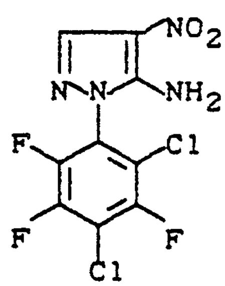

(Verfahren e)

5,0 g (0.0177 Mol) 5-Amino-1-(2,4-dichlor-3,5,6-trifluorphenyl)-pyrazol in 40 ml 96 %iger Schwefelsäure gibt man tropfenweise unter Rühren und Kühlung bei 0 °C bis 5 °C zu einer Mischung aus 0,17 g (0.0028 Mol) Harnstoff und 1,45 ml (0.034 Mol) 98 %iger Salpetersäure in 40 ml 96 %iger Schwefelsäure. Nach beendeter Zugabe rührt man eine weitere Stunde bei 0 °C bis 5 °C gibt dann die Reaktionsmischung in 400 ml Eiswasser, filtriert den kristallinen Niederschlag ab, wäscht mit Wasser neutral und trocknet im Vakuum bei 50 °C.

Man erhält 5,5 g (95 % der Theorie) an 5-Amino-1-(2,4-dichlor-3,5,6-trifluorphenyl)-4-nitropyrazol vom Schmelzpunkt 169 °C - 173 °C.

In entsprechender Weise und gemäß den allgemeinen Angaben zur Herstellung erhält man die folgenden 5-Amino-1-phenylpyrazole der allgemeinen Formel (I),

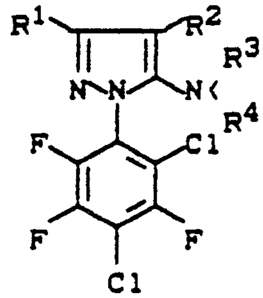

(I)

Tabelle 1

| Bsp.-Nr. | $R^1$ | $R^2$ | 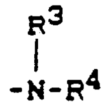 | Schmelzpunkt (°C) |
|---|---|---|---|---|
| 6 | H | H | 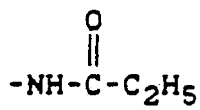 | 140 - 144 |
| 7 | H | $NO_2$ | 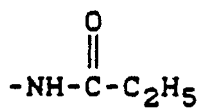 | 133 - 137 |
| 8 | H | H | 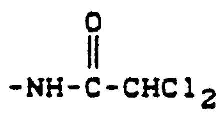 | 115 - 117 |
| 9 | H | $NO_2$ | 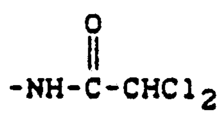 | 123 - 127 |
| 10 | H | H | 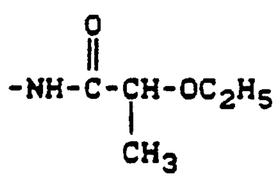 | 111 - 114 |
| 11 | H | $NO_2$ | 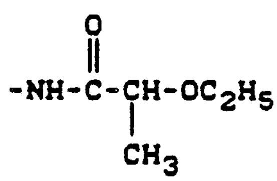 | 96 - 98 |

Tabelle 1 (Fortsetzung)

| Bsp.-Nr. | $R^1$ | $R^2$ | $-\overset{R^3}{\overset{\vert}{N}}-R^4$ | Schmelzpunkt (°C) |
|---|---|---|---|---|
| 12 | H | H | $-NH-\overset{O}{\overset{\Vert}{C}}-CH_2-CH_2-Cl$ | 125 - 127 |
| 13 | H | $NO_2$ | $-NH-\overset{O}{\overset{\Vert}{C}}-CH_2-CH_2-Cl$ | 119 - 123 |
| 14 | H | $NO_2$ | $-NH-\overset{O}{\overset{\Vert}{C}}-CH_2-CH_2-OCH_3$ | 113 - 116 |
| 15 | H | H | $-NH-\overset{O}{\overset{\Vert}{C}}-CH_2-CH_2-OCH_3$ | $^1H$-NMR*): 7.75 |
| 16 | H | H | $-NH-\overset{O}{\overset{\Vert}{C}}-\underset{CH_3}{\underset{\vert}{CH}}-Cl$ | $^1H$-NMR*): 7.76 |
| 17 | H | $NO_2$ | $-NH-\overset{O}{\overset{\Vert}{C}}-\underset{CH_3}{\underset{\vert}{CH}}-Cl$ | 108 - 112 |
| 18 | H | $NO_2$ | $-NH-\overset{O}{\overset{\Vert}{C}}-\underset{C_2H_5}{\underset{\vert}{CH}}-Cl$ | $^1H$-NMR*): 8.28 |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindung

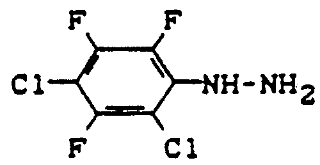

Zu 500 g (2,28 Mol) 2,4,5,6-Tetrafluor-1,3-dichlorbenzol (vgl. z. B. US-PS 3 574 775 oder US-PS 3 852 365) in 1100 ml Ethanol gibt man 140 g (2,8 Mol) Hydrazinhydrat, erhitzt anschließend für 4 Stunden auf Rückflußtemperatur, engt im Vakuum ein, verrührt den Rückstand mit 500 ml Wasser, saugt den so erhaltenen Niederschlag ab, trocknet ihn und kristallisiert aus Cyclohexan um.

Man erhält 450 g (85 % der Theorie) an 2,4-Dichlor-3,5,6-trifluorphenylhydrazin vom Schmelzpunkt 80 °C -81 °C.

Anwendungsbeispiele:

In den folgenden Anwendungsbeispielen wurde die nachstehend aufgeführte Verbindung als Vergleichssubstanz eingesetzt:

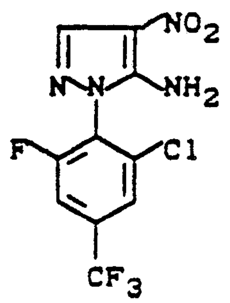

(A)

5-Amino-1-(2-chlor-6-fluor-4-trifluormethylphenyl)-4-nitro-pyrazol
(bekannt aus DE-OS 34 02 308).

Beispiel A

Pre-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % | = keine Wirkung (wie unbehandelte Kontrolle) |
| 100 % | = totale Vernichtung |

Eine deutliche Überlegenheit in der Nutzpflanzenselektivität gegenüber der Vergleichssubstanz (A) zeigen in diesem Test z. B. die Verbindungen gemäß den Herstellungsbeispielen: (5), (7), (9), (11) und (14).

Beispiel B

Post-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % | = keine Wirkung (wie unbehandelte Kontrolle) |
| 100 % | = totale Vernichtung |

Eine deutliche Überlegenheit in der Nutzpflanzenselektivität gegenüber der Vergleichssubstanz (A) zeigen in diesem Test z. B. die Verbindungen gemäß den Herstellungsbeispielen: (5), (7), (9) und (11).

## Ansprüche

1. 5-Amino-1-phenylpyrazole der Formel (I),

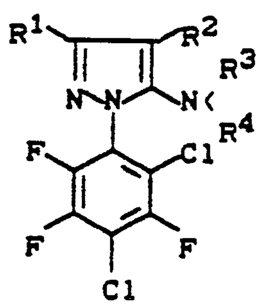

(I)

in welcher

$R^1$ für Wasserstoff oder Alkyl steht,

$R^2$ für Wasserstoff, Nitro, Hydroxycarbonyl oder Alkoxycarbonyl steht,

$R^3$ für Wasserstoff, Alkyl, Alkenyl, Alkinyl, Halogenalkyl, Halogenalkenyl, Halogenalkinyl oder für einen Rest $-\underset{\underset{X}{\|}}{C}-R^5$

steht,

$R^4$ für Wasserstoff, Alkyl, Alkenyl, Alkinyl, Halogenalkyl, Halogenalkenyl, Halogenalkinyl oder für einen Rest $-\underset{\underset{X}{\|}}{C}-R^5$ steht, wobei

$R^5$ für Wasserstoff, Alkyl, Alkenyl, Alkinyl, Halogenalkyl, Halogenalkenyl, Alkoxyalkyl, Alkylthioalkyl, Alkylsulfonylalkyl, Alkylsulfinylalkyl, für gegebenenfalls substituiertes Cycloalkyl, für jeweils gegebenenfalls substituiertes Aryloxyalkyl oder Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und

X für Sauerstoff oder Schwefel steht,

2. 5-Amino-1-phenylpyrazole der Formel (I) gemäß Anspruch 1,
in welcher

$R^1$ für Wasserstoff oder für geradkettiges oder verzweigtes Alkyl mit 1 bis 6 Kohlenstoffatomen steht,

$R^2$ für Wasserstoff, Nitro, Hydroxycarbonyl oder geradkettiges oder verzweigtes Alkoxycarbonyl mit 1 bis 6 Kohlenstoffatomen steht,

$R^3$ für Wasserstoff, für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halogenatomen oder für einen Rest $-\underset{\underset{X}{\|}}{C}-R^5$ steht,

$R^4$ für Wasserstoff, für jeweils geradkettiges oder verzweigtes Alkyl mit 1 bis 8 Kohlenstoffatomen, Alkenyl mit 3 bis 8 Kohlenstoffatomen, Alkinyl mit 3 bis 8 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Halogenalkinyl mit 3 bis 6 Kohlenstoffatomen und 1 bis 9 gleichen oder verschiedenen Halgenatomen oder für einen Rest $-\underset{\underset{X}{\|}}{C}-R^5$ steht, wobei

$R^5$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 6 Kohlenstoffatomen, für jeweils geradkettiges oder verzweigtes Halogenalkyl mit 1 bis 4 Kohlenstoffatomen und 1 bis 9 gleichen oder verschiedenen Halogenatomen oder Halogenalkenyl mit 2 bis 6 Kohlenstoffatomen und 1 bis 9 gleichen oder verschiedenen Halogenatomen oder für jeweils geradkettiges oder verzweigtes Alkoxyalkyl, Alkylthioalkyl, Alkylsulfonylalkyl, Alkylsulfinylalkyl, Alkoxy, Alkylthio, Alkylamino oder Dialkylamino mit jeweils 1 bis 4 Kohlenstoffatomen in den einzelnen Alkylteilen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenoxymethyl, Phenoxyethyl, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen oder Halogen alkyl mit 1 bis 4 Kohlenstoffatomen und 1 bis 9 gleichen oder verschiedenen Halogenatomen und

X für Sauerstoff oder Schwefel steht.

3. 5-Amino-1-phenylpyrazole der Formel (I) gemäß Anspruch 1
in welcher

$R^1$ für Wasserstoff, Methyl, Ethyl, n- oder i-Propyl sowie n-, i-, s- oder t-Butyl steht,

$R^2$ für Wasserstoff, Nitro, Hydroxycarbonyl, Methoxycarbonyl oder Ethoxycarbonyl steht,

$R^3$ für Wasserstoff, Methyl, Ethyl, n- oder i-Propyl, n-, i-, s- oder t-Butyl, für Allyl, n- oder i-Butenyl, Propargyl, n- oder i-Butinyl, für Chlorethyl, Trifluorethyl, Bromethyl, für Chlorallyl, Dichlorallyl, Brompropargyl oder für einen Rest $-\underset{\underset{X}{\|}}{C}-R^5$ steht und

$R^4$ für Wasserstoff, Methyl, Ethyl, n- oder i-Propyl, n-, i-, s- oder t-Butyl, für Allyl, n- oder i-Butenyl, Progargyl, n- oder i-Butinyl, für Chlorethyl, Trifluorethyl, Bromethyl, für Chlorallyl, Dichlorallyl, Brompropargyl oder für einen Rest $-\underset{\underset{X}{\|}}{C}-R^5$ steht, wobei

$R^5$ für Wasserstoff, Methyl, Ethyl, n- oder i-Propyl, n-, i-, s- oder t-Butyl, jeweils geradkettiges oder

verzweigtes Pentyl, Hexyl, Heptyl, Octyl, Nonyl, Decyl oder Undecyl, Vinyl, Allyl, Chlorallyl, Propargyl, Butenyl, Methoxymethyl, Ethoxymethyl, Methoxyethyl, Ethoxyethyl, Methylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methoxy, Ethoxy, n- oder i-Propoxy, Methylthio, Ethylthio, Methylamino, Ethylamino, Dimethylamino, Diethylamino, Trifluormethyl, Trichlormethyl, Dichlorfluormethyl, Difluorchlormethyl, Chlormethyl, Iodmethyl, Brommethyl, Dichlormethyl, Difluormethyl, 1-Chlorethyl, 2-Chlorethyl, 1-Brommethyl, 2-Bromethyl, 1-Chlorpropyl, 3-Chlorpropyl, Heptafluorpropyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl oder für jeweils gegebenenfalls ein- bis dreifach, gleich oder verschieden durch Methyl, Methoxy, Fluor, Chlor oder Trifluormethyl substituiertes Phenyl, Phenoxymethyl, Phenoxy, Phenylthio oder Phenylamino steht und

X für Sauerstoff und Schwefel steht.

4. Verfahren zur Herstellung von 5-Amino-1-phenylpyrazolen der Formel (I)

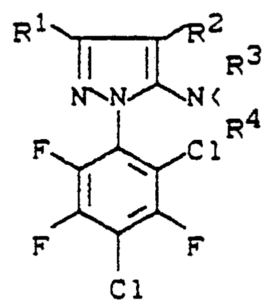

(I)

in welcher

$R^1$ für Wasserstoff oder Alkyl steht,

$R^2$ für Wasserstoff, Nitro, Hydroxycarbonyl oder Alkoxycarbonyl steht,

$R^3$ für Wasserstoff, Alkyl, Alkenyl, Alkinyl, Halogenalkyl, Halogenalkenyl, Halogenalkinyl oder für für einen Rest $-\underset{X}{\overset{\|}{C}}-R^5$ steht,

$R^4$ für Wasserstoff, Alkyl, Alkenyl, Alkinyl, Halogenalkyl, Halogenalkenyl, Halogenalkinyl oder für einen Rest $-\underset{X}{\overset{\|}{C}}-R^5$ steht, wobei

$R^5$ für Wasserstoff, Alkyl, Alkenyl, Alkinyl, Halogenalkyl, Halogenalkenyl, Alkoxyalkyl, Alkylthioalkyl, Alkylsulfonylalkyl, Alkylsulfinylalkyl, für gegebenenfalls substituiertes Cycloalkyl, für jeweils gegebenenfalls substituiertes Aryloxyalkyl oder Aryl, für Alkoxy, Alkylthio, für gegebenenfalls substituiertes Aryloxy, für gegebenenfalls substituiertes Arylthio, für Alkylamino, Dialkylamino oder für gegebenenfalls substituierte Arylamino steht und

X für Sauerstoff oder Schwefel steht,

dadurch gekennzeichnet, daß man

(a) 5-Amino-1-phenylpyrazole der Formel (Ia),

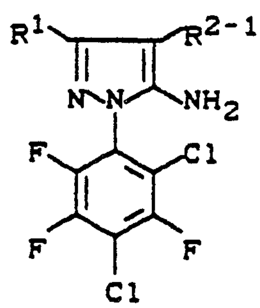

(Ia)

in welcher

$R^{2-1}$ für Wasserstoff, Alkoxycarbonyl oder Nitro steht und
$R^1$ die oben angegebene Bedeutung hat,
erhält, wenn man 2,4-Dichlor-3,5,6-trifluorphenylhydrazin der Formel (II)

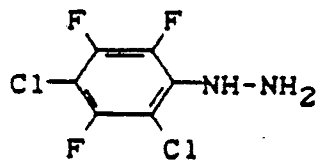
(II)

(α) mit Acrylnitril-Derivaten der Formel (IIIa),

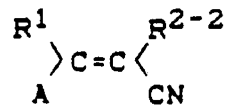
(IIIa)

in welcher
$R^{2-2}$ für Alkoxycarbonyl oder Nitro steht,
A für Halogen, Hydroxy, Alkoxy, Alkylamino oder Dialkylamino steht und
$R^1$ die oben angegebene Bedeutung hat oder
(β) mit 2-Chloracrylnitrilen der Formel (IIIb),

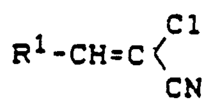
(IIIb)

in welcher
$R^1$ die oben angegebene Bedeutung hat,
zunächst in einer 1. Stufe gegebenenfalls in Gegenwart eines Verdünnungsmittels und gegebenenfalls in Gegenwart eines Reaktionshilfsmittels umsetzt zu den Phenylhydrazinderivaten der Formel (IV),

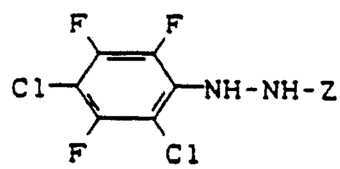
(IV)

in welcher
Z für einen Rest

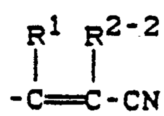

oder für einen Rest

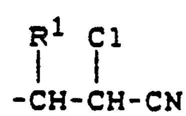

steht, wobei $R^1$ und $R^{2-2}$ die oben angegebene Bedeutung haben,

und diese in einer 2. Stufe gegebenenfalls in Gegenwart eines Verdünnungsmittels und gegebenenfalls in Gegenwart eines Reaktionshilfsmittels cyclisiert; oder daß man

(b) 5-Amino-1-phenylpyrazole der Formel (Ib),

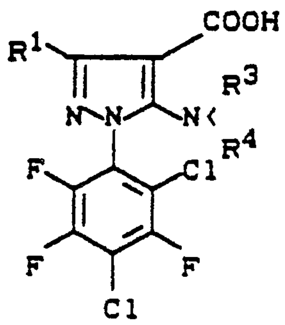

(Ib)

in welcher
$R^1$, $R^3$ und $R^4$ die oben angegebene Bedeutung haben,
erhält, wenn man 5-Amino-1-phenylpyrazole der Formel (Iz),

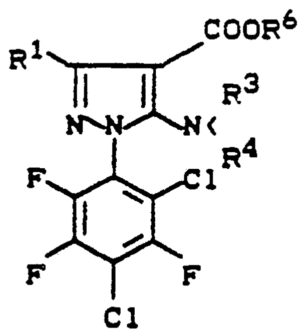

(Iz)

in welcher
$R^6$ für Alkyl steht und
$R^1$, $R^3$ und $R^4$ und oben angegebene Bedeutung haben,
an der Estergruppe in 4-Position des Pyrazolringes gegebenenfalls in Gegenwart eines Verdünnungsmittels und gegebenenfalls in Gegenwart eines Reaktionshilfsmittels verseift; oder daß man

(c) 5-Amino-1-phenylpyrazole der Formel(Ic),

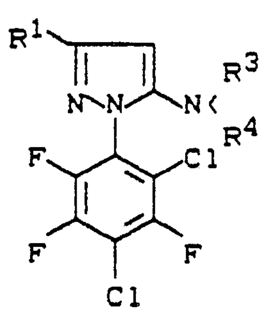

(Ic)

in welcher
$R^1$, $R^3$ und $R^4$ die oben angegebene Bedeutung haben,
erhält, wenn man 5-Amino-1-phenylpyrazole der Formel (Ib),

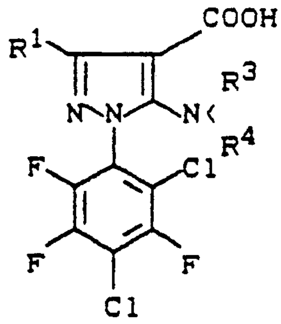

(Ib)

in welcher
$R^1$, $R^3$ und $R^4$ die oben angegebene Bedeutung haben,
gegebenenfalls in Gegenwart eines Verdünnungsmittels und gegebenenfalls in Gegenwart eines Katalysators decaboxyliert; oder daß man

d) 5-Amino-1-phenylpyrazole der Formel (Id),

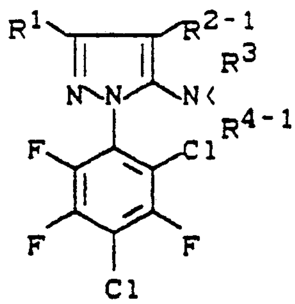

(Id)

in welcher
$R^{2-1}$ für Wasserstoff, Alkoxycarbonyl oder Nitro steht,
$R^{4-1}$ für Alkyl, Alkenyl, Alkinyl, Halogenalkyl, Halogenalkenyl, Halogenalkinyl oder für einen Rest $-\underset{\underset{X}{\|}}{C}-R^5$
steht und
$R^1$, $R^3$, $R^5$ und X die oben angegebene Bedeutung haben,
erhält, wenn man 5-Amino-1-phenylpyrazole der Formel (Iy),

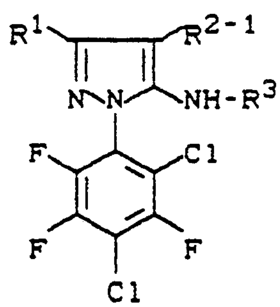

(Iy)

in welcher
$R^1$, $R^{2-1}$ und $R^3$ die oben angegebene Bedeutung haben,
($\alpha$) mit Acylierungsmitteln der Formel (V),

$$R^5-\overset{\overset{X}{\|}}{C}-A^1 \qquad (V)$$

in welcher
$A^1$ für eine elektronenanziehende Abgangsgruppe steht und
$R^5$ und X die oben angegebene Bedeutung haben, oder
($\beta$) mit Alkylierungsmitteln der Formel (VI),

$R^{4-2}$-$A^2$ (VI)

in welcher

$R^{4-2}$ für Alkyl, Alkenyl, Alkinyl, Halogenalkyl, Halogenalkenyl oder Halogenalkinyl steht und

$A^2$ für eine elektronenanziehende Abgangsgruppe steht,

jeweils gegebenenfalls in Gegenwart eines Verdünnungsmittels und gegebenenfalls in Gegenwart eines Reaktionshilfsmittels usmetzt; oder daß man

(e) 5-Amino-1-phenylpyrazole der Formel (Ie),

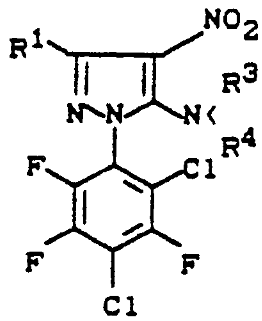

(Ie)

in welcher

$R^1$, $R^3$ und $R^4$ die oben angegebene Bedeutung haben,

erhält, wenn man 5-Amino-1-phenylpyrazole der Formel (Ic),

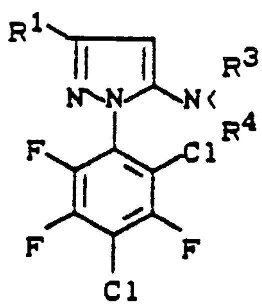

(Ic)

in welcher

$R^1$, $R^3$ und $R^4$ die oben angegebene Bedeutung haben,

mit Nitrierungsmitteln gegebenenfalls in Gegenwart eines Verdünnungsmittels und gegebenenfalls in Gegenwart eines Reaktionshilfsmittels umsetzt; oder daß man

(f) 5-Amino-1-phenylpyrazole der Formel (If),

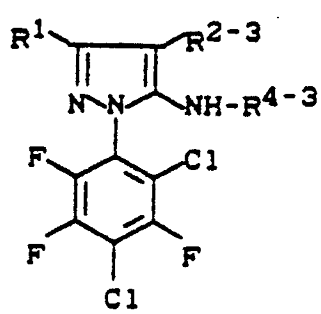

(If)

in welcher

$R^{2-3}$ für Wasserstoff oder Nitro steht,

$R^{4-3}$ für Wasserstoff, Alkyl, Alkenyl, Alkinyl, Halogenalkyl, Halogenalkenyl oder Halogenalkinyl steht und

$R^1$ die oben angegebene Bedeutung hat,

erhält, wenn man 5-Amino-1-phenylpyrazole der Formel (Ix),

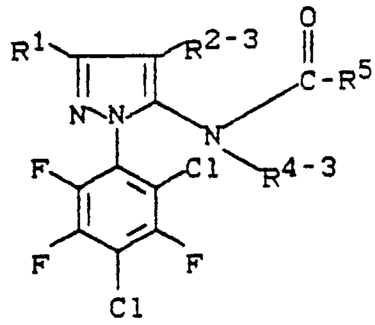

(Ix)

in welcher
$R^1$, $R^{2-3}$, $R^{4-3}$ und $R^5$ die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes deacyliert; oder daß man

(g) 5-Amino-1-phenylpyrazole der Formel (Ig),

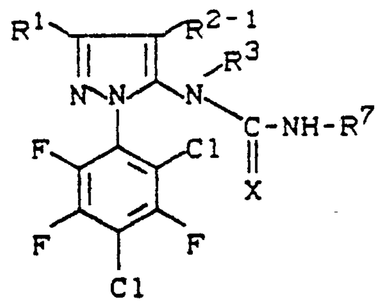

(Ig)

in welcher
$R^{2-1}$ für Wasserstoff, Alkoxycarbonyl oder Nitro steht,
$R^7$ für Alkyl oder für gegebenenfalls substituiertes Aryl steht und
$R^1$, $R^3$ und X die oben angegebene Bedeutung haben,
erhält, wenn man 5-Amino-1-phenylpyrazole der Formel (Iy),

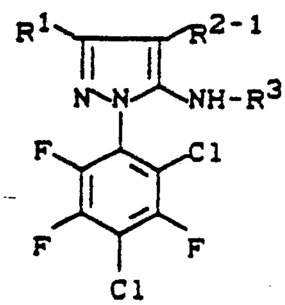

(Iy)

in welcher
$R^1$, $R^{2-1}$ und $R^3$ die oben angegebene Bedeutung haben,
mit Iso(thio)cyanaten der Formel (VII),

$R^7$-N=C=X (VII)

in welcher
$R^7$ und X die oben angegebene Bedeutung haben,
gegebenenfalls in Gegenwart eines Verdünnungsmittels und gegebenenfalls in Gegenwart eines Reaktionshilfsmittels umsetzt; oder daß man

(h) 5-Amino-1-phenylpyrazole der Formel (Ih),

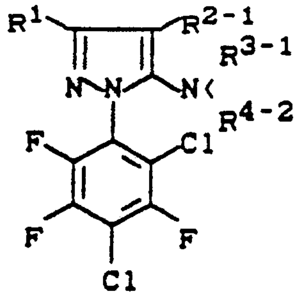

(Ih)

in welcher
$R^{2-1}$ für Wasserstoff, Alkoxycarbonyl oder Nitro steht,
$R^{3-1}$ für Wasserstoff, Alkyl, Alkenyl, Alkinyl, Halogenalkyl, Halogenalkenyl oder Halogenalkinyl steht,
$R^{4-2}$ für Alkyl, Alkenyl, Alkinyl, Halogenalkyl, Halogenalkenyl oder Halogenalkinyl steht und
$R^1$ die oben angegebene Bedeutung hat,
erhält, wenn man 5-Halogenpyrazole der Formel (VIII),

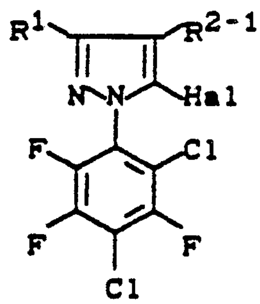

(VIII)

in welcher
Hal für Halogen steht und
$R^1$ und $R^{2-1}$ die oben angegebene Bedeutung haben,
mit Aminen der Formel (IX),

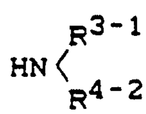

(IX)

in welcher
$R^{3-1}$ und $R^{4-2}$ die oben angegebene Bedeutung haben,
gegebenenfalls in Gegenwart eines Verdünnungsmittels und gegebenenfalls in Gegenwart eines Reaktionshilfsmittels umsetzt; oder daß man

(i) 5-Amino-1-phenylpyrazole der Formel (Ii),

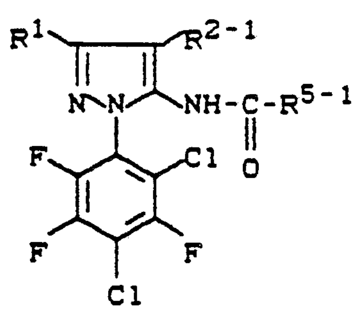

(Ii)

in welcher
$R^{2-1}$ für Wasserstoff, Alkoxycarbonyl oder Nitro steht,

$R^{5-1}$ für Alkoxy, Alkylthio, Alkylamino oder Dialkylamino oder für jeweils gegebenenfalls substituiertes Aryloxy, Arylthio oder Arylamino steht und
$R^1$ die oben angegebene Bedeutung hat,
erhält, wenn man (Bis)Carbamate der Formel (Iw),

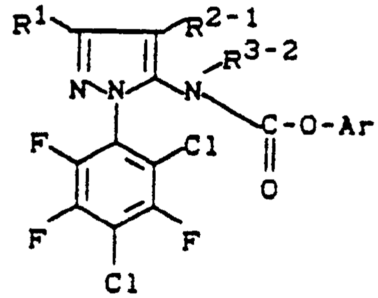

(Iw)

in welcher
$R^{3-2}$ für Wasserstoff oder für einen Rest $-\underset{\underset{O}{\|}}{C}-O-Ar$
steht, wobei
Ar für gegebenenfalls substituiertes Aryl steht und
$R^1$ und $R^{2-1}$ die oben angegebene Bedeutung haben,
mit Alkoholen, Aminen oder Thiolen der Formel (X),
$R^{5-1}$-H (X)
in welcher
$R^{5-1}$ die oben angegebene Bedeutung hat,
gegebenenfalls in Gegenwart eines Verdünnungsmittels und gegebenenfalls in Gegenwart eines Reaktionshilfsmittels umsetzt.

5. Herbizide Mittel, gekennzeichnet durch einen Gehalt an mindestens einem 5-Amino-1-phenylpyrazol der Formel (I) gemäß den Ansprüchen 1 bis 4.

6. Verfahren zur Bekämpfung von Unkräutern, dadurch gekennzeichnet, daß man 5-Amino-1-phenylpyrazole der Formel (I) gemäß den Ansprüchen 1 bis 4 auf die Unkräuter und/oder ihren Lebensraum einwirken läßt.

7. Verwendung von 5-Amino-1-phenylpyrazolen der Formel (I) gemäß den Ansprüchen 1 bis 4 zur Bekämpfung von Unkräutern.

8. Verfahren zur Herstellung von herbiziden Mitteln, dadurch gekennzeichnet, daß man 5-Amino-1-phenylpyrazole der Formel (I) gemäß den Ansprüchen 1 bis 4 mit Streckmitteln und/oder oberflächenaktiven Substanzen vermischt.

9. 5-Halogenpyrazole der Formel (VIII),

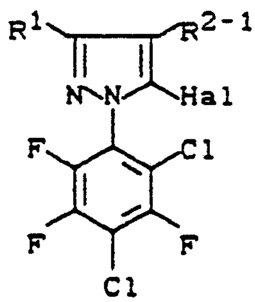

(VIII)

in welcher
$R^1$ für Wasserstoff oder Alkyl steht,
$R^{2-1}$ für Wasserstoff, Alkoxycarbonyl oder Nitro steht und
Hal für Halogen steht.

10. Verfahren zur Herstellung von 5-Halogenpyrazolen der Formel (VIII)

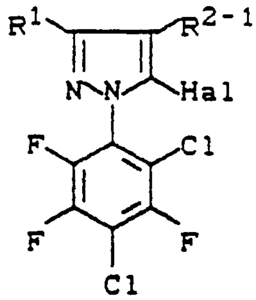

(VIII)

in welcher
$R^1$ für Wasserstoff oder Alkyl steht,
$R^{2-1}$ für Wasserstoff, Alkoxycarbonyl oder Nitro steht und
Hal für Halogen steht,
dadurch gekennzeichnet, daß man
Alkoxymethylenmalonester der Formel (XI),

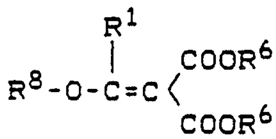

(XI)

in welcher
$R^1$ die oben angegebene Bedeutung hat und
$R^6$ und $R^8$ unabhängig voneinander jeweils für Alkyl stehen,
mit 2,4-Dichlor-3,5,6-trifluorphenylhydrazin der Formel (II)

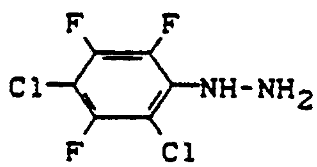

(II)

zunächst in einer ersten Stufe gegebenenfalls in Gegenwart eines Verdünnungsmittels umsetzt zu den so erhältlichen Pyrazolcarbonsäureester der Formel (XII),

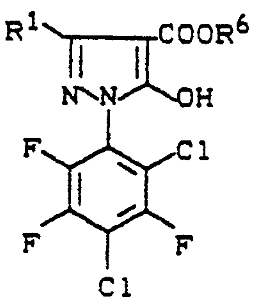

(XII)

in welcher
$R^1$ und $R^6$ die oben angegebene Bedeutung haben,
diese gegebenenfalls in einer zwischengeschalteten 2. Stufe gegebenenfalls in Gegenwart eines Verdünnungsmittels und gegebenenfalls in Gegenwart einer Base verseift und decarboxyliert zu Pyrazolinonen der Formel (XIII),

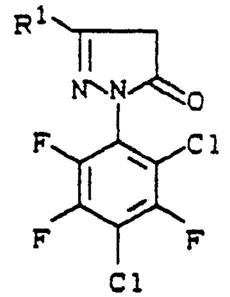

(XIII)

in welcher
$R^1$ die oben angegebene Bedeutung hat,
und entweder die Pyrazolcarbonsäureester der Formel (XII) oder die Pyrazolinone der Formel (XIII) in einer 3. Stufe mit Halogenierungsmitteln umsetzt, und gegebenenfalls in einer 4. Stufe die so erhältlichen 5-Halogen-pyrazole der Formel (VIIIa),

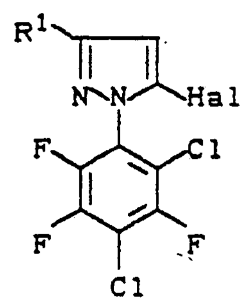

(VIIIa)

in welcher
$R^1$ und Hal die oben angegebene Bedeutung haben,
mit Nitrierungsmitteln gegebenenfalls in Gegenwart eines Verdünnungsmittels und gegebenenfalls in Gegenwart eines Reaktionshilfsmittels oder Katalysators in 4-Stellung des Pyrazolringes nitriert.

Europäisches Patentamt

# EUROPÄISCHER RECHERCHENBERICHT

Nummer der Anmeldung

EP 89104340.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A2/A3 - 0 224 831 (BAYER AG)<br>* Ansprüche 1,3-8 *<br>-- | 1-8 | C 07 D 231/38<br>C 07 D 231/40<br>C 07 D 231/16<br>A 01 N 43/56 |
| A | WO - A1 - 87/03 781 (MAY & BAKER LTD)<br>* Anspruch 1 *<br>---- | 1-10 | |
| | | | RECHERCHIERTE SACHGEBIETE (Int. Cl.4) |
| | | | C 07 D 231/00 |

Der vorliegende Recherchenbericht wurde für alle Patentansprüche erstellt.

| Recherchenort | Abschlußdatum der Recherche | Prüfer |
|---|---|---|
| WIEN | 05-06-1989 | BRUS |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82